# EUROPEAN PATENT APPLICATION

(11) **EP 2 093 222 A1**
(43) Date of publication of application: **26.08.2009**
(21) Application number: 08380055.7
(22) Date of filing: 22.02.2008
(51) Int. Cl.: C07D 303/04, C07D 303/12, C07D 303/38, C07D 407/06, A61K 31/336

(54) **Polyisoprenoid epoxides useful for decreasing cholesterol and/or increasing coenzyme Q biosynthesis**

(71) Applicant: NEWBIOTECHNIC, S.A., 41110 Bollullos de la Mitacion (Sevilla) (ES); Universidad Pablo de Olavide, 41013 Sevilla (ES); University of Stockholm & Hudding Hospital, Solna, 17176 Stockholm (SE)
(72) Inventor: Navas Lloret, Plácido, 41013 Sevilla (ES); Dallner, Gustav, 171 76 Stockholm (SE); Rey Barrera, Manuel, 41110 Bollullos de la Mitación, Sevilla (ES)
(74) Representative: ABG Patentes, S.L.

(57) **Abstract**

Polyisoprenoid epoxides having *all-trans* double bonds are capable to stimulate the synthesis of coenzyme Q and/or inhibit the synthesis of cholesterol, thereby being useful in the treatment of diseases related to high cholesterol levels, and/or a CoQ deficiency.

## Description

### FIELD OF THE INVENTION

The present invention relates to polyisoprenoid epoxides and their stereoisomers, derivatives, solvates and salts thereof, to processes for producing said compounds and to their uses. Said polyisoprenoid epoxides stimulate the synthesis of coenzyme Q and/or inhibit the synthesis of cholesterol, thereby being useful in the treatment of several diseases.

### BACKGROUND OF THE INVENTION

Arteriosclerosis and heart infarct are among the most relevant diseases of the Western world. It is generally accepted and proved that the main reason behind said diseases is high blood cholesterol. This lipid is an important constituent of the body and required for normal functioning. One-third of the necessary cholesterol is taken up from the diet and two-thirds are synthesized in the body, mainly in the liver. If the blood cholesterol is above normal level, cholesterol is deposited in the arteries and the arteriosclerotic process is initiated.

In order to counteract the disease and the consequent destruction of the arteries, one has to decrease the cholesterol concentration in the blood. The easiest way to do this is to decrease the dietary cholesterol, leading to a decreased uptake from the intestine to the blood stream. This dietary restriction is, however, efficient only when there is an initial moderate level of lipids in blood, but in the large majority of patients the dietary restriction does not normalize blood cholesterol levels.

Thus, inhibition of cholesterol synthesis is of considerable medical interest and, at present, the statins, inhibitors of HMG-CoA reductase (an enzyme involved in the initial steps of cholesterol and coenzyme Q10 biosynthesis), are employed almost exclusively for this purpose. Statins have been used worldwide during more than twenty years and it is apparent that said drugs, in most cases, efficiently decrease blood cholesterol levels. However, said drugs have serious and unfavourable side effects, such as damage to muscle and kidney functions. A further drawback of statin treatment is that it also interfers with CoQ biosynthesis and protein isoprenylation [Liao, J. K. (2005) Curr Opin Lipidol 16(6), 624-629; Littarru, G. P., and Langsjoen, P. (2007) Mitochondrion 7 Suppl, S168-174]. Therefore, effective inhibition of cholesterol biosynthesis downstream from the branch-point represented by famesyl-PP synthase might prove to be highly beneficial.

Coenzyme Q (CoQ) is a coenzyme formed by a biologically active quinine having a polyisoprenoid side-chain comprising several isoprene units. There are different types of CoQ which can be distinguished by the number of isoprene units contained in the side-chain, e.g., CoQ6, which contains 6 isoprene units and can be found in *Saccharomyces cerevisiae,* CoQ8, which contains 8 isoprene units and can be found in *Escherichia coli,* CoQ9, which contains 9 isoprene units and can be found in *Caenorhabditis elegans,* etc. In human beings, the most common form of CoQ is CoQ10 (which contains 10 isoprene units in the side-chain), also known as ubiquinone, although significant amounts of CoQ are also produced.

CoQ is present in the membranes of all animal cells, where it performs a number of essential functions. In addition to the roles it plays in the mitochondrial respiratory chain and as the only lipid-soluble antioxidant synthesized endogenously, CoQ also participates in extra-mitochondrial electron transport, functional modification of mitochondrial uncoupling proteins, regulation of the mitochondrial permeability transition pore and modulation of the levels of certain receptors on the surface of blood monocytes [Ernster, L. and Dallner, G. (1995) Biochim Biophys Acta 1271(1), 195-204; Bentinger, M. et al. (2007) Mitochondrion 7 Suppl, S41-50]. Furthermore, CoQ influences the expression of a large number of genes whose products are involved in a number of metabolic processes [Groneberg, D. A. et al. (2005) Int J Biochem Cell Biol 37(6), 1208-1218; Doring, F. et al. (2007) IUBMB Life 59(10), 628-633].

Under a variety of conditions, the tissue level of CoQ is reduced, an alteration that is considered to exert a negative impact of cellular functions. During aging, the levels of this lipid in all organs of the rat and human are severely decreased. Moreover, diseases such as cardiomyopathy, neuropathy, nefropathy, muscle degeneration and cancer (e.g., liver cancer) are associated with significantly lower tissue levels of CoQ [Littarru, G. P. et al. (1996) Clinical aspects of Coenzyme Q: Improvement of cellular bioenergetics or antioxidant protection? In: Handbook of Antioxidants, Marcel Dekker, New York, pp 203-239]. In addition, genetic disorders characterized by impaired biosynthesis of CoQ involve serious metabolic disturbances that can be partially counteracted by dietary supplementation. However, to date, the only way to increase the CoQ content is by dietary supplementation. Nevertheless, CoQ is taken up very poorly from the intestines and only 2% of the dietary lipid appears in the blood. Additionally, only some but not all organs take up this lipid from the blood. Consequently, in the case of a CoQ deficiency there are very limited treatment possibilities and in most cases said deficiency cannot be remedied. Under such conditions, elevating tissue levels of CoQ would be beneficial.

The biosynthesis of cholesterol is influenced by a number of endogenous metabolites, including oxysterols, squalene oxide, famesol and its derivatives, geranylgeraniol, prenyl phosphates and sterols that activate meiosis. It appears highly probable that as-yet-unidentified metabolites also regulate CoQ biosynthesis. For instance, in connection with diseases in which oxidative stress is a contributing factor, such as Alzheimer's and prion diseases, precarcinogenic conditions and diabetes, biosynthesis of CoQ is up-regulated [Turunen, M. et al. (2004) Biochim Biophys Acta 1660, 171-199].

The relatively short half-life of CoQ in tissues, varying between 50 and 125 hours, leads to the formation of significant amounts of metabolites, some of which may be regulators of the biosynthesis of this lipid. Degradation products isolated from the urine and feces of rats and guinea pigs contain an intact and fully substituted ring together with a short, carboxylated side-chain. These compounds are subject to glucuronidation or phosphorylation prior to excretion into the bile and urine, respectively. Moreover, various breakdown products of CoQ may be produced in association with UV irradiation and lipid peroxidation. Thus, UV irradiation of skin rapidly reduces its content of CoQ, while such irradiation of crystals of this lipid yields a number of degradation products. In its reduced form, CoQ in submitochondrial particles and liposomes is protected from lipid peroxidation, but the oxidized form is subject to extensive breakdown to more polar, as-yet-unidentified compounds [Forsmark-Andree, P. et al. (1997) Free Radic Biol Med 22(3), 391-400].

Approaches designed to enhance endogenous synthesis of CoQ are of considerable interest, since dietary supplementation is inefficient, with only a few percent of this lipid being taken up from the intestinal tract and transferred from the blood into the various organs. Unlike the situation with respect to cholesterol, no metabolic regulators of CoQ biosynthesis have yet been identified, even though this synthesis is known to be up-regulated under certain conditions. In rodents, but not in humans, agonists of the nuclear receptor PPARα, including clofibrate, di(2-ethylhexyl)phthalate and acetylsalicylic acid, both stimulate the biosynthesis of CoQ and elevate the level of this lipid in all organs, with the exception of the brain (Aberg, F. et al. (1994) Chem Biol Interact 91(1), 1-14; Turunen, M. et al. (2000) J Mol Biol 297(3), 607-614). Among the hormones, thyroxine induces hepatic biosynthesis of CoQ and may be responsible for the elevated level of this lipid in the livers of rodents maintained at 4°C for 10 days (Aithal, H. N. et al. (1968) Biochim Biophys Acta 162(1), 66-72; Ikeda, S. et al. (1984) Horm Metab Res 16(11), 585-588). Furthermore, both vitamin A deficiency and inhibition of squalene synthase by squalestatin 1 lead to elevated levels of CoQ in both cultured cells and rats (Thelin, A. et al. (1994) Biochim Biophys Acta 1215(3), 245-249; Keller, R. K. (1996) Biochim Biophys Acta 1303(3), 169-179).

There is a need, therefore, for drugs that increase CoQ synthesis and/or decrease cholesterol synthesis. In particular, drugs that decrease cholesterol synthesis and increase or at least maintain CoQ synthesis could be useful not only for treatment of patients with a high level of blood cholesterol but also for treatment of patients with diseases where CoQ content in blood and tissues is low or has been decreased. This problem is solved by the compounds of the present invention.

### SUMMARY OF THE INVENTION

In the search for compounds that up-regulate the biosynthesis of CoQ, inventors surprisingly discovered that irradiation of CoQ with ultraviolet (UV) light results in the formation of a number of compounds that influence the synthesis of mevalonate pathway lipids by HepG2 cells. Among the compounds that potently stimulated CoQ synthesis while inhibiting cholesterol synthesis, derivatives of CoQ containing 1-4 epoxide moieties in their polyisoprenoid side-chains were identified. Subsequently, chemical epoxidation of *all-trans* polyprenols of different lengths revealed that solanesol epoxides enhanced CoQ and markedly reduced cholesterol biosynthesis. In contrast, none of the modified *trans-trans-polycis* polyprenols exerted noticeable effects. Tocotrienol epoxides were especially potent in the system used by the inventors: those with one epoxide moiety in the side-chain generally up-regulated CoQ biosynthesis by 200-300%, while those with two such moieties also decreased cholesterol synthesis by 50-90%. Thus, by varying the number of epoxide moieties on the polyisoprene chain, differential effects on CoQ and cholesterol synthesis can be achieved. Further, prolonged treatment of HepG2 cells with tocotrienol epoxides for 26 days elevated their content of CoQ by 30%. In addition, the levels of mRNA encoding enzymes involved in CoQ biosynthesis were also elevated by the tocotrienol epoxides.
The site of inhibition of cholesterol synthesis was shown to be oxidosqualene cyclase. Consequently, epoxide derivatives of certain *all-trans* polyisoprenoids cause pronounced stimulation of CoQ synthesis and, in some cases, simultaneous reduction of cholesterol biosynthesis by HepG2 cells.

Therefore, the inventors have surprisingly found that epoxide derivatives of all-*trans* polyisoprenoids can stimulate CoQ synthesis, and, in some cases, simultaneously reduce cholesterol synthesis by HepG2 cells, thus said compounds being capable of increasing CoQ levels and/or decreasing cholesterol levels efficiently. This is the basis for the development of a drug which can be administered to humans and other animals in order to interfere with a disturbed lipid metabolism. Said compounds can be easily obtained from *all-trans* polyisoprenoid lipids, which can be found in various types of biological materials such as bacteria, yeast, plant and animal tissues.

Thus, in an aspect, the present invention relates to an epoxide derivative of an *all-trans* polyisoprenoid, its stereoisomers, derivatives, solvates and salts, including its pharmaceutically acceptable salts thereof. In a particular embodiment, said compound is capable to stimulate CoQ synthesis thus increasing CoQ levels efficiently. In another particular embodiment, said compound is capable to inhibit cholesterol synthesis thus decreasing cholesterol levels efficiently. In another particular embodiment, said compound is capable of both to stimulate CoQ synthesis and to inhibit cholesterol synthesis thus increasing CoQ levels and decreasing cholesterol levels efficiently. In another particular embodiment, said compound is capable to inhibit cholesterol synthesis and to stimulate, or at least maintain, CoQ synthesis thus decreasing cholesterol levels and increasing, or at least maintaining, CoQ levels efficiently.

In other aspect, the invention relates to a process for obtaining said epoxide derivative of an all-*trans* polyisoprenoid, its stereoisomers, derivatives, solvates and salts, including its pharmaceutically acceptable salts thereof. The composition obtained through said process constitutes a further aspect of this invention.

In another aspect, the invention relates to said epoxide derivative of an all-*trans* polyisoprenoid, its stereoisomers, derivatives, solvates and pharmaceutically acceptable salts, for treating a disease related to high cholesterol levels, and/or a CoQ deficiency.

In another aspect, the invention relates to the use of said epoxide derivative of an all-*trans* polyisoprenoid, its stereoisomers, derivatives, solvates and pharmaceutically acceptable salts, for the manufacture of a pharmaceutical composition, specially, for use in the manufacture of a pharmaceutical composition for treating a disease related to high cholesterol levels and/or a disease based on a CoQ deficiency.

In another aspect, the invention relates to a process for the manufacture of a pharmaceutical composition for treating a disease related to high cholesterol levels and/or a disease based on a CoQ deficiency, wherein the compound acting as the main active component is said epoxide derivative of an all-*trans* polyisoprenoid, its stereoisomers, derivatives, solvates and pharmaceutically acceptable salts.

In another aspect, the invention relates to a pharmaceutical composition comprising said epoxide derivative of an all*-trans* polyisoprenoid, its stereoisomers, derivatives, solvates and pharmaceutically acceptable salts, and a pharmaceutically acceptable excipient.

### BRIEF DESCRIPTION OF THE DRAWINGS

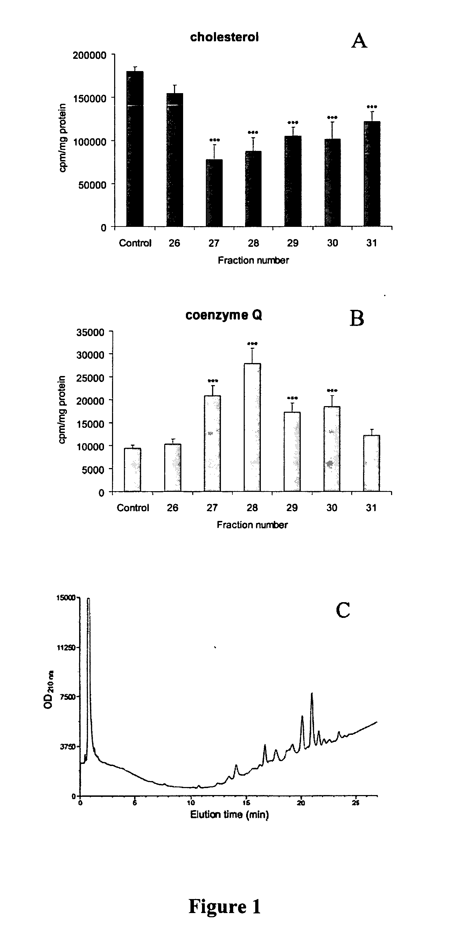

Figure 1 shows the effects of compounds produced by UV irradiation of CoQ₁₀ on the biosynthesis of cholesterol and CoQ. A solution of CoQ₁₀ in ethanol:water (9:1) was irradiated with UV light, followed by fractionation on a silica gel column. Aliquots of the fractions thus obtained (50 µg/10 ml medium) were added to cultures of HepG2 cells, followed 24 hrs later by the addition of [³H]mevalonate (0.5 mCi). After 8 additional hrs of culturing, the cells were harvested by trypsinisation and their lipids extracted and analyzed by HPLC employing on-line detection of radioactivity. A) The level of radioactivity recovered in cholesterol; Control = non-treated cells, 26-31 = collected fractions. B) Radioactivity in CoQ; Control = non-treated cells, 26-31 = collected fractions. C) The HPLC chromatogram of fraction 28. These values are the means ± SE (vertical bars) of 4 independent experiments. ****P*<0.001.

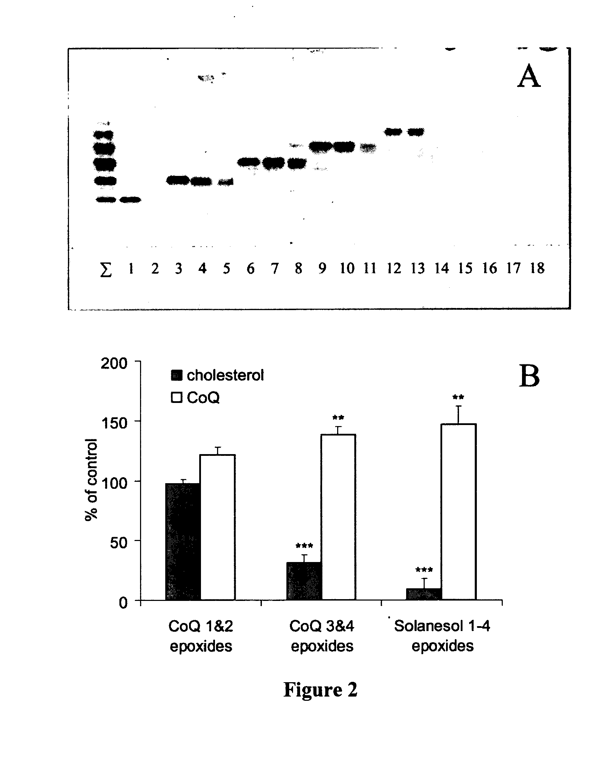

Figure 2 shows the results of the chemical epoxidation of CoQ₁₀ and solanesol and the effects of these epoxide derivatives on cholesterol and CoQ synthesis. A) CoQ₁₀ was epoxidated with 3-chloroperoxybenzoic acid in dichloromethane, the products separated by chromatography on silica gel and the individual fractions thus collected analyzed by chromatography on silica gel-60 plates using acetone:methanol (2: 1) as the developing solvent system. E = the total reaction mixture; fractions eluted from the silica column: 1 = unreacted CoQ; 3-5 = CoQ containing one epoxide moiety; 6-8 = CoQ with 2 epoxide moieties; 9-11 = CoQ with 3 epoxide moieties; 12-13 = CoQ with 4 epoxide moieties. B) HepG2 cells were cultured in the presence of derivatives of CoQ containing 1-4 epoxide moieties or of solanesol with 1-4 epoxide moieties at a final concentration of 2 µM. After 24 hrs of treatment, the culture medium was supplemented with 0.5 mCi [³H]mevalonate and 8 hrs later the cells were harvested by trypsinisation and their lipids extracted and analyzed by HPLC using on-line detection of radioactivity. The filled columns represent cholesterol, the empty columns CoQ. These values (given as percentages of the corresponding control values) are the means ± SE (vertical bars) of three independent experiments. ***P*<0.01, ****P*<0.001.

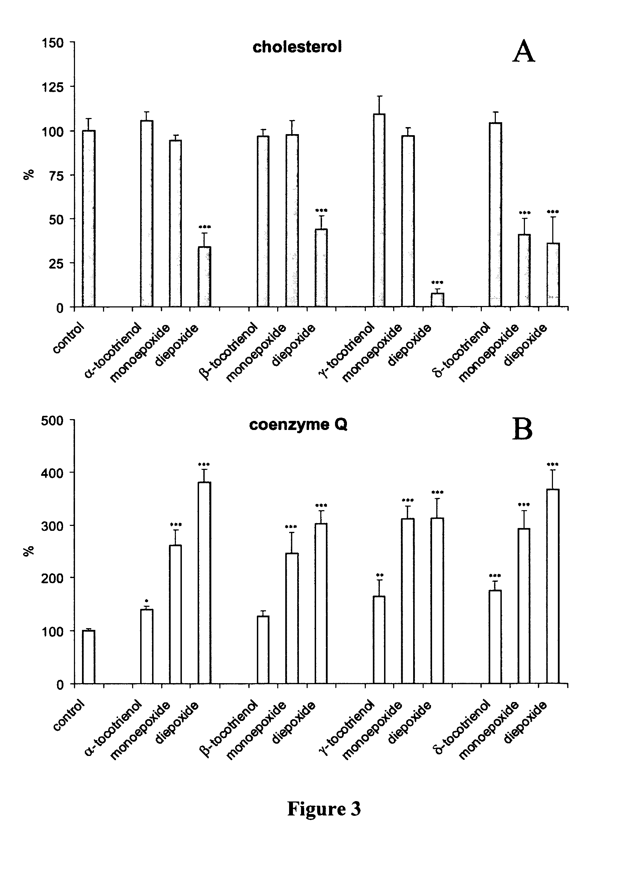

Figure 3 shows the effects of tocotrienol epoxides on cholesterol and CoQ biosynthesis. α-, β-, γ- and δ-tocotrienols were epoxidated and the resulting compounds containing one or two epoxide moieties then separated by column chromatography on silica gel. The parent and epoxidated products were incubated with HepG2 cells at a final concentration of 5 µM for 24 hrs, followed by supplementation with 0.5 mCi [³H]mevalonate and incubation for an additional 8 hrs. At this point, lipids were extracted from the cells and analyzed by HPLC. A) Radioactive labeling of cholesterol; B) Labeling of CoQ₁₀. The values presented are the means ± SE (the vertical bars) of 4 independent experiments. **P*<0.05, ***P*<0.01, ****P*<0.001.

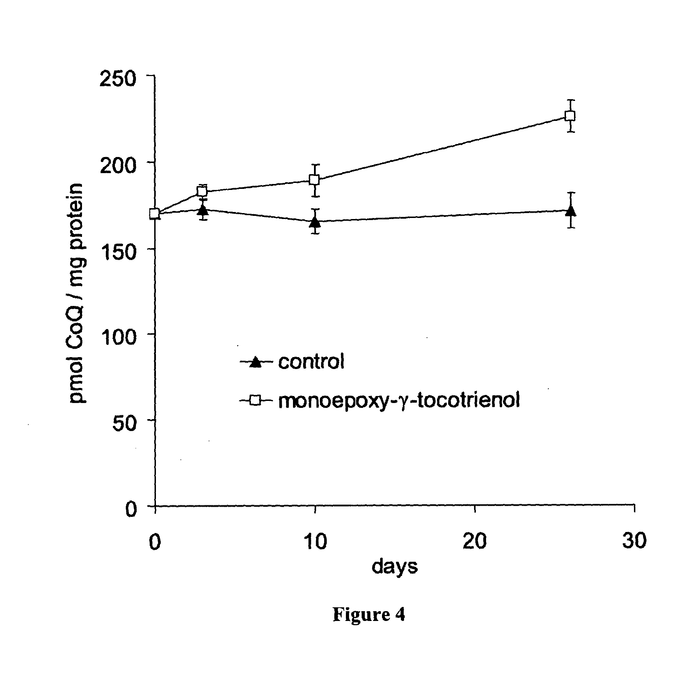

Figure 4 shows the effects of prolonged treatment with monoepoxy-γ-tocotrienol on the level of CoQ in HepG2 cells. HepG2 cells cultured in the presence of 5 µM monoepoxy-γ-tocotrienol were split following 3, 6, 10, 15 and 20 days of culturing. Following 3, 10 and 26 days, the CoQ content of these cells was determined by lipid extraction and subsequent HPLC. Filled triangels = control, untreated cells; empty squares = monoepoxy-γ-tocotrienol-treated cells. These values are means ± SE (vertical bars) (n = 4). **P*<0.05, ***P*<0.01, ****P*<0.001.

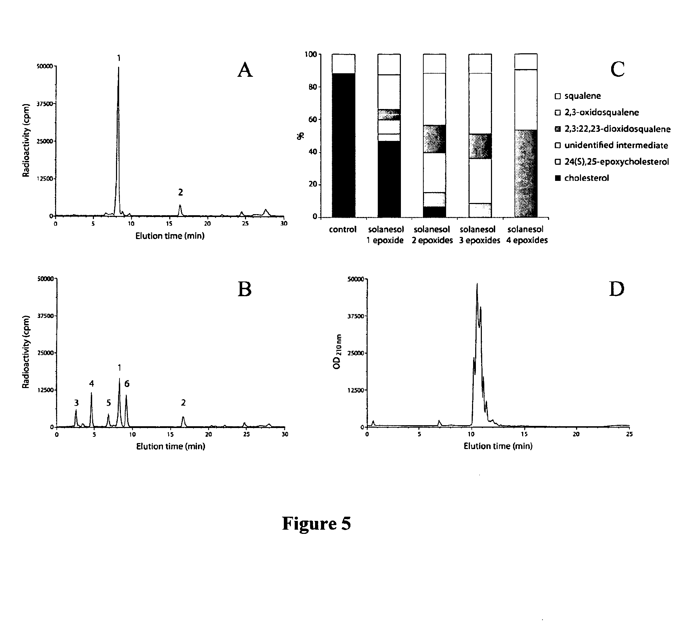

Figure 5 shows the inhibition of cholesterol synthesis by solanesol epoxides. HepG2 cells were cultured in the presence of 2 µM diepoxysolanesol for 24 hrs and thereafter labeled with [³H]mevalonate for 8 hrs. Subsequently, cellular lipids were extracted and analyzed by HPLC. A) In control cells, cholesterol is labeled (1) and the only intermediate observed is squalene (2). B) Cells cultured in the presence of the diepoxysolanesol exhibit reduced labeling of cholesterol (1) and five additional radioactive peaks are seen, namely, squalene (2), 24(S),25-epoxycholesterol (3), 2,3:22,23-dioxidosqualene (4), an unidentified intermediate (5) and 2,3-oxidosqualene (6). C) The distribution of cholesterol and its biosynthetic intermediates in HepG2 cells treated with 2 µM solanesol derivatives possessing 1-4 epoxide moieties. The individual columns illustrate the relative amounts in terms of percentages of the total amount of radioactivity recovered. D) The HPLC chromatogram of diepoxysolanesol reveals the presence of a complex mixture, reflecting the hetorogenous localization of the epoxides within the molecule.

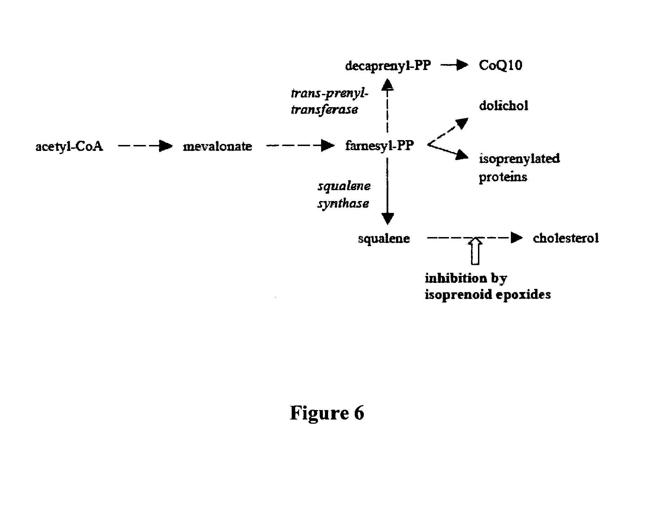

Figure 6 is a schematic representation of the mevalonate pathway.

### DETAILED DESCRIPTION OF THE INVENTION

### Definitions

As used in the specification and appended claims, unless otherwise specified, the following terms have the meaning indicated below.

"Acyl" refers to a radical of formula Ra-C(=O), wherein Ra is alkyl or aryl as defined herein.

"Alkyl" refers to a straight or branched chain monovalent or divalent radical consisting solely of carbon and hydrogen, containing no unsaturation and having from 1 to 12 carbon atoms, e.g., methyl, ethyl, n-propyl, 1-methylethyl (iso-propyl), n-butyl, n-pentyl, 1,1-dimethylethyl (t-butyl), n-heptyl, etc. Unless otherwise stated specifically in the specification, the term "alkyl" is meant to include alkyl radicals which are optionally substituted by one or more substituents independently selected from the group consisting of acyl, alkoxy, alkoxycarbonyl, alkylthio, amino, aryl, carboxy, carbonyl, cyano, halogen, hydroxy, imino, mercapto and nitro, e.g., -O-propyl, -O-benzyl (OBn), O-benzoyl (OBz), etc. In a particular embodiment, "alkyl" refers to a straight or branched chain monovalent or divalent radical consisting solely of carbon and hydrogen, containing no unsaturation and having from 1 to 6 carbon atoms.

"Alkenyl" refers to a straight or branched chain monovalent or divalent radical consisting solely of carbon and hydrogen, containing at least one double bond and having from 2 to 12 carbon atoms, e.g., ethenyl, prop-1-enyl, but-1-enyl, pent-1-enyl, penta-1,4-dienyl, etc. The double bond of an alkenyl radical may be unconjugated or conjugated to another unsaturated group (if present). Unless otherwise stated specifically in the specification, the term "alkenyl" is meant to include alkenyl radicals which are optionally substituted by one or more substituents independently selected from the group consisting of acyl, alkoxy, alkoxycarbonyl, alkylthio, amino, aryl, carboxy, carbonyl, cyano, halogen, hydroxy, imino, mercapto and nitro, e.g., -O-propyl, -O-benzyl (OBn), O-benzoyl (OBz), etc. In a particular embodiment, "alkenyl" refers to a straight or branched chain monovalent or divalent radical consisting solely of carbon and hydrogen, containing at least one double bond and having from 2 to 6 carbon atoms.

"Alkoxy" refers to a radical of formula Ra-O-, wherein Ra is alkyl as defined herein.

"Alkoxycarbonyl" refers to a radical of formula -C(O)ORa, wherein Ra is alkyl as defined herein, e.g., methoxycarbonyl, ethoxycarbonyl, n-propoxycarbonyl, and the like.

"Alkylthio" refers to a radical of formula -S-Ra where Ra is an alkyl radical as defined above, e.g., methylthio, ethylthio, n-propylthio, and the like.

"Alkynyl" refers to a straight or branched chain monovalent or divalent radical consisting solely of carbon and hydrogen, containing at least one triple bond and having from 2 to 12 carbon atoms, e.g., ethynyl, propynyl (e.g. 1-propynyl, 2-propynyl), butynyl (e.g. 1-butynyl, 2-butynyl, 3-butynyl), pentynyl (e.g. 1-pentynyl, 2-pentynyl, 3-pentynyl, 4-pentynyl,), hexynyl (e.g. 1-hexynyl, 2-hexynyl, 3-hexynyl, 4-hexynyl, 5-hexynyl,), methylpropynyl, 3-methyl-1-butynyl, 4-methyl-2-heptynyl, 4-ethyl-2-octynyl, etc. The triple bond of an alkynyl radical may be unconjugated or conjugated to another unsaturated group (if present). Unless otherwise stated specifically in the specification, the term "alkynyl" is meant to include alkynyl radicals which are optionally substituted by one or more substituents independently selected from the group consisting of acyl, alkoxy, alkoxycarbonyl, alkylthio, amino, aryl, carboxy, carbonyl, cyano, halogen, hydroxy, imino, mercapto and nitro, e.g., -O-propyl, -O-benzyl (OBn), O-benzoyl (OBz), etc. In a particular embodiment, "alkynyl" refers to a straight or branched chain monovalent or divalent radical consisting solely of carbon and hydrogen, containing at least one triple bond and having from 2 to 6 carbon atoms.

"Amino" refers to the radical -N(Rc)(Rd), wherein Rc and Rd, independently, Rc and Rd are H, alkyl or aryl, as defined herein.

"Aryl" refers to an aromatic hydrocarbon radical having at least 6 carbon atoms, e.g., 6 to 14 carbon atoms, such as phenyl, naphthyl or anthracyl. The aryl radical may be optionally substituted by one or more substituents such as acyl, alkyl, alkoxy, alkoxycarbonyl, amino, aminoalkyl, cyano, haloalkyl, halogen, hydroxy, mercapto, nitro, phenyl, etc., as defined herein.

"Aryl-hydrogenated" refers to total or partially hydrogenated derivatives of an aryl radical as defined herein. Unless otherwise stated specifically in the specification, the term "aryl-hydrogenated" is meant to include total or partially hydrogenated cyclic radicals having at least 6 carbon atoms, e.g., 6 to 14 carbon atoms, including condensed total or partially hydrogenated cyclic radicals, such as octahydronaphtyl, decahydroanthracyl, etc. The aryl-hydrogenated radical may be optionally substituted by one or more substituents such as acyl, alkyl, alkoxy, alkoxycarbonyl, amino, aminoalkyl, cyano, haloalkyl, halogen, hydroxy, mercapto, nitro, phenyl, etc., as defined herein.

"Aryloxy" refers to a radical of formula Rb-O-, wherein Rb is aryl as defined herein.

"Aryloxycarbonyl" refers to a radical of formula -C(O)ORb, wherein Rb is aryl as defined herein, e.g., benzyloxycarbonyl, etc.
"Carbonyl" refers to a group of formula C(=O).
"Carboxy" refers to a group of formula -COOH.
"Cyano" refers to the radical -CN.
"Cycloalkyl" refers to a stable 3- to 12-membered monocyclic or bicyclic radical which is saturated, and which consist solely of carbon and hydrogen atoms, e.g., cyclopropyl, cyclobutyl, cyclobutyl, cyclohexyl, decalinyl, etc. Unless otherwise stated specifically in the specification, the term "cycloalkyl" is meant to include cycloalkyl radicals which are optionally substituted by one or more substituents independently selected from the group consisting of acyl, alkoxy, alkoxycarbonyl, alkyl, alkylthio, amino, aryl, carboxy, carbonyl, cyano, halogen, hydroxy, imino, mercapto and nitro, e.g., phenyl, etc.

The term "derivative" as used herein means a chemical compound having undergone a chemical derivation such as substitution or addition of a further chemical group to change (for pharmaceutical use) any of its physico-chemical properties, such as solubility or bioavailability. Derivatives include so-called prodrugs, e.g. ester and ether derivatives of an active compound that yield the active compound *per se* after administration to a subject. Preferred examples of derivatives are succinyl or acetyl derivatives, attached to hydroxy groups.

The term "excipient" refers to a carrier or vehicle, in general, with which the active ingredient [polyisoprenoid epoxides of the invention] is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as excipients, particularly for injectable solutions. Suitable pharmaceutical excipients are described in "Remington's Pharmaceutical Sciences" by E.W. Martin.

"Halogen" means -F, -Cl, -Br or -I.

"Heterocyclyl" refers a radical derived from a heterocycle by removal of an atom of hydrogen from any ring atom, namely to a stable 3- to 15-membered ring which consists of carbon atoms and from 1 to 15 heteroatoms selected from the group consisting of nitrogen, oxygen, and sulphur, preferably a 4- to 8-membered ring with one or more heteroatoms, more preferably a 5- or 6-membered ring with one or more heteroatoms. For the purposes of this invention, the heterocycle may be a monocyclic, bicyclic or tricyclic ring system, which may include fused ring systems; the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.
"Hydroxy" refers to the radical -OH.
"Imine" refers to a radical containing a carbon-nitrogen double bond.
"Mercapto" refers to the radical -SH.
"Nitro" refers to the radical -NO₂.

"Polyisoprenoid", as used herein, a polyisoprenoid is a compound derived from isoprene (2-methylbuta-1,3-diene), the skeleton of which can generally be discerned in repeated occurrence in the molecule. As it is known, the skeleton of isoprenoids may differ from strict additivity of isoprene units by loss or shift of a fragment, commonly a methyl group. The term "polyisoprenoid" includes both hydrocarbons (e.g., terpenes, etc.) and oxygenated derivatives (e.g., polyprenols, etc.), as well as derivatives thereof such as the acetylated, phosphorylated, phtalimidated, etc. thereof. The polyisoprenoids may be, or not, bound to an aromatic system. The conformation of the double bonds which are present in said polyisoprenoids may be *cis* or *trans*; in fact, there are polyisoprenoids identified as: all-cis polyisoprenoids, i.e., polyisoprenoids having only *cis* residues (i.e., the conformation of all the double bonds which are present in said compounds is cis) and *all-trans* polyisoprenoids, i.e., polyisoprenoids having only *trans* residues (i.e., the conformation of all the double bonds which are present in said compounds is *trans*); and polyisoprenoids having cis residues and *trans* residues. A polyisoprenoid for use in the present invention is an *all-trans* polyisoprenoid which comprises (i) at least, 2 isoprene units; (ii) at least, one double bond; and (iii) the conformation of all the double bonds which are present in said compound is *trans.* In a particular embodiment, a polyisoprenoid for use in the present invention comprises: (i) a variable number of isoprene units, e.g., from 2 to 100, preferably from 2 to 80, more preferably from 2 to 61 isoprene units; (ii) a variable number of double bonds, e.g., from 1 to 100, typically from 1 to 80, preferably from 1 to 60, more preferably from 1 to 40, still more preferably from 1 to 20, even still more preferably from 1 to 10 double bonds; and (iii) the conformation of all the double bonds which are present in said compound is *trans.* Further, the polyisoprenoid may have a saturated or unsaturated alpha end and/or a saturated or unsaturated omega end. Further, the polyisoprenoid may be bound to an organic moiety, typically a substituted or unsubstituted aromatic system or a total or partial hydrogenated form thereof. Illustrative, non-limitative examples of polyisoprenoids which can be used for producing the polyisoprenoid epoxides of the invention include polyprenols, such as farnesol, geraniol, geranylgeraniol, solanesol, etc., terpenes, e.g., squalene, etc., as well as polisoprenoids bound to aromatic systems, such as vitamin K₂, CoQ, tocotrienols, etc.

The term "salt" refers to any salt of the polyisoprenoid epoxides of the invention, including both, pharmaceutically acceptable salts and non-pharmaceutically acceptable salts. The term "pharmaceutically acceptable salts" refers to any salt, which, upon administration to the recipient is capable of providing (directly or indirectly) a polyisoprenoid epoxide of the invention as described herein. Non-pharmaceutically acceptable salts also fall within the scope of the invention since they may be useful in the preparation of pharmaceutically acceptable salts. The preparation of the salts can be carried out by methods known in the art. For instance, pharmaceutically acceptable salts as well as non-pharmaceutically acceptable salts of the polyisoprenoids of the invention may be acid addition salts, base addition salts or metallic salts, and they can be synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the base addition salts include inorganic salts such as, for example, ammonium, and organic base salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium, aluminium and lithium salts. Further, the term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "stereoisomer" as used herein makes reference to compounds made up of the same atoms bonded by the same sequence of bonds but having different three-dimensional structures which are not interchangeable.

The term "therapeutically effective amount" refers to the quantity of active ingredient calculated to produce the desired effect and will generally be determined, among other reasons, by the own features of the active ingredient used and the therapeutic effect to be obtained. In a particular embodiment, the dose of active ingredient administered to a subject in need of treatment for the treatment and/or prophylaxis of the above mentioned conditions is within the range of 10⁻¹⁰ to 10¹⁰ mg/kg of body weight, typically between 10⁻³ and 10³ mg/kg of body weight.

References herein to substituted groups in the compounds of the invention refer to the specified moiety that may be substituted at one or more available positions by one or more suitable groups, e. g., an alkyl as defined above, halogen such as fluoro, chloro, bromo and iodo; cyano; hydroxyl; nitro; azido; -C(=O)OH;-C(=O)OR"; -C(=O)NH₂; - C(=O)NHR"; -C(=O)NR"R"'; -C(=O)R"; alkenyl and alkynyl groups including groups having one or more unsaturated linkages and from 2 to about 12 carbon or from 2 to about 6 carbon atoms; -OR"; -OAr, such as phenoxy; -SR"; alkylsulfinyl (alkylS(=O)-) groups including those moieties having one or more sulfinyl linkages and from 1 to about 12 carbon atoms or from 1 to about 6 carbon atoms; alkylsulfonyl groups including those moieties having one or more sulfonyl linkages and from 1 to about 12 carbon atoms or from 1 to about 6 carbon atoms; -NH₂; -NHR"; -NR"R"' or aryl; wherein R" and R"' are independently selected from alkyl or alkenyl radical as defined above. Unless otherwise indicated, an optionally substituted group may have a substituent at each substitutable position of the group, and each substitution is independent of the other.

### Polyisoprenoid epoxide of the invention

In an aspect, the invention relates to an epoxide derivative of an all-*trans* polyisoprenoid or a derivative thereof, hereinafter referred to as "polyisoprenoid epoxide of the invention". The term "polyisoprenoid epoxide of the invention" as used herein further includes the stereoisomers, derivatives, solvates and salts of the polyisoprenoid epoxides of the invention. The polyisoprenoid epoxide of the invention may be obtained (is obtainable) by a process which comprises the full or partial oxidation of an *all-trans* polyisoprenoid.

According to the invention, the polyisoprenoid epoxide of the invention comprises (i) at least, 2 isoprene units; (ii) at least, 1 epoxide moiety; (iii) at least, 1 double bond; and (iv) the conformation of all the double bonds which are present in said polyisoprenoid epoxide is *trans.* Thus, in a particular embodiment, the polyisoprenoid epoxide of the invention comprises: (i) a variable number of isoprene units, e.g., from 2 to 100, preferably from 2 to 80, more preferably from 2 to 61 isoprene units; (ii) a variable number of epoxide moieties, e.g., from 1 to 100, typically from 1 to 80, preferably from 1 to 60, more preferably from 1 to 40, still more preferably from 1 to 20, even still more preferably from 1 to 10 epoxide moieties; (iii) a variable number of double bonds, e.g., from 1 to 100, typically from 1 to 80, preferably from 1 to 60, more preferably from 1 to 40, still more preferably from 1 to 20, even still more preferably from 1 to 10 double bonds; and (iv) the conformation of all the double bonds which are present in said compound is *trans.* Further, the polyisoprenoid epoxide of the invention may have a saturated or unsaturated alpha end and/or a saturated or unsaturated omega end. Illustrative, non-limitative examples of polyisoprenoids which can be used for producing the polyisoprenoid epoxides of the invention include polyprenols, such as farnesol, geraniol, geranylgeraniol, solanesol, etc., terpenes, e.g., squalene, etc., as well as polisoprenoids bound to aromatic systems, such as vitamin K₂, CoQ, tocotrienols, etc.

As mentioned above, the number of isoprene units which can be present in the polyisoprenoid epoxide of the invention can vary within a broad range. In a particular embodiment, the number of isoprene units ranges from 2 to 100, preferably from 2 to 80, more preferably from 2 to 61 isoprene units, still more preferably from 2 to 30, even still more preferably from 2 to 15, usually from 2 to 10 isoprene units; in a preferred embodiment the number of isoprene units is 2, 3, 4, 5, 6, 7, 8, 9 or 10.

The number of epoxide moieties which can be present in the polyisoprenoid epoxide of the invention is at least 1 and can also vary within a broad range. In a particular embodiment, the number of epoxide moieties ranges from 1 to 100, typically from 1 to 80, preferably from 1 to 60, more preferably from 1 to 40, still more preferably from 1 to 20, even still more preferably from 1 to 10 epoxide moieties; in a preferred embodiment, the number of epoxide moieties is 1, 2, 3, 4, 5, 6, 7, 8, 9 or 10. In general, the number of epoxide moieties within the polyisoprenoid epoxide of the invention will typically depend, among other things, on the number of double bonds which are present within the *all-trans* polyisoprenoid compound used as starting material. Thus, the famesol epoxide may contain 1, 2 or 3 epoxide moieties, e.g., 1 or 2; the geranylgeraniol epoxide may contain 1, 2, 3 or 4 epoxide moieties, e.g., 1, 2 or 3; the solanesol epoxide may contain up to 9 epoxide moieties, e.g., 1, 2, 3, 4, 5, 6, 7, 8, or 9, preferably 1, 2, 3, or 4; the squalene epoxide may contain 1, 2, 3, 4, 5 or 6 epoxide moieties, e.g., 4 or less, such as 3 or less; the CoQ epoxide may contain from 1 to 10 epoxide moieties, e.g., 9 or less, 8 or less, 6 or less; the tocotrienols epoxides may contain 1, 2 or 3 epoxide moieties; etc.

The number of double bonds which can be present in the polyisoprenoid epoxide of the invention is at least 1 and can also vary within a broad range. In a particular embodiment, the number of double bonds ranges from 1 to 100, typically from 1 to 80, preferably from 1 to 60, more preferably from 1 to 40, still more preferably from 1 to 20, even still more preferably from 1 to 10 double bonds; in a preferred embodiment the number of double bonds is 1, 2, 3, 4, 5, 6, 7, 8, 9 or 10. In practice the number of double bonds may not correspond with the number of isoprene units because the double bond originally contained in any of said isoprene units may be saturated. Further, the conformation of all the double bonds which are present in said polyisoprenoid epoxide of the invention is *trans.*

Additionally, the polyisoprenoid epoxide of the invention may have a saturated or unsaturated alpha end as well as a saturated or unsaturated omega end.

In a particular embodiment, the polyisoprenoid epoxide of the invention is a compound of formula (I)

R-(X)ₙ-(Y)ₘ-Z (I)

wherein 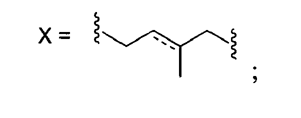 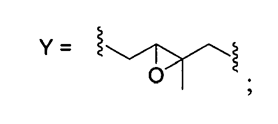 X and Y and can be randomly alternated; 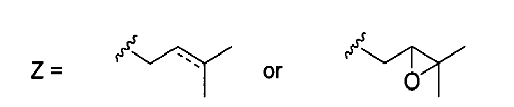 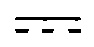 represents an optionally saturated or unsaturated carbon-carbon bond;
n + m is comprised between 1 and 60, wherein n is an integer from 0 to 59 and m is an integer from 1 to 60, and the repeated units can be independently saturated or unsaturated; and
R is selected from the group consisting of H, OH, R¹OC(O)-,
R¹OP(=O)₂O-, a radical of formula (A), a radical of formula (B) or a radical of formula (C) 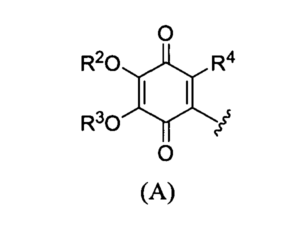 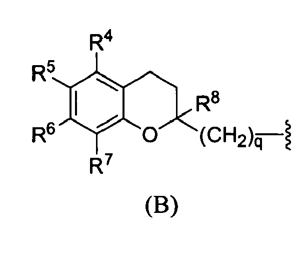 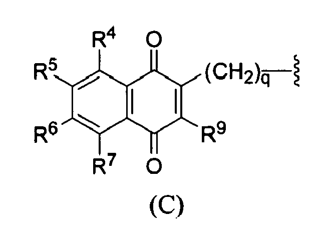 wherein

R¹, R² and R³, independently, are selected from the group consisting of hydrogen, alkyl, cycloalkyl, alkenyl, alkynyl, aryl, aryl-hydrogenated and heterocyclyl; and, optionally, R² and R³ together form (i) a straight or branched divalent radical consisting only of carbon and hydrogen atoms containing no unsaturation and having from 2 to 10 carbon atoms; or (ii) a straight or branched unsaturated divalent radical consisting only of carbon and hydrogen atoms having one or more double bonds and/or one or more triple bonds and having from 2 to 10 carbon atoms, wherein said double or triple bonds can be unconjugated or conjugated to another double or triple bonds unsaturated group, said divalent radicals (i) and (ii) being optionally substituted with one or more substituents selected from halogen, hydroxy, acyl, alkoxy, alkoxycarbonyl, aryloxy, aryloxycarbonyl, carbonyl, carboxy, cyano, amino, imino, nitro, mercapto or alkylthio;

R⁴, R⁵, R⁶, R⁷ and R⁸, independently, are selected from the group consisting of hydrogen, hydroxyl, alkyl, cycloalkyl, alkenyl, alkynyl, aryl, aryl-hydrogenated, heterocyclyl, cyano, -COR¹⁰, -C(O)OR¹⁰, -C(O)NR¹⁰R¹¹, - C=NR¹⁰, -OR¹⁰, OC(O)R¹⁰, -S(O)ₚR¹⁰ -NR¹⁰R¹¹, -NR¹⁰C(O)R¹¹, -NO₂, - N=CR¹⁰R¹¹ or halogen; and, optionally, R⁵ and R⁶ together form (i) a straight or branched divalent radical consisting only of carbon and hydrogen atoms containing no unsaturation and having from 2 to 10 carbon atoms; or (ii) a straight or branched unsaturated divalent radical consisting only of carbon and hydrogen atoms having one or more double bonds and/or one or more triple bonds and having from 2 to 10 carbon atoms, wherein said double or triple bonds can be unconjugated or conjugated to another double or triple bonds unsaturated group, said divalent radicals (i) and (ii) being optionally substituted with one or more substituents selected from halogen, hydroxy, acyl, alkoxy, alkoxycarbonyl, aryloxy, aryloxycarbonyl, carbonyl, carboxy, cyano, amino, imino, nitro, mercapto or alkylthio; wherein R¹⁰ and R¹¹, independently, are selected from hydrogen, alkyl, cycloalkyl, alkenyl, alkinyl, aryl, aryl-hydrogenated, heterocyclyl, alkoxy or aryloxy;
R⁹ is hydrogen or alkyl;
p can take the value 0, 1 or 2; and
q is 0 or 1;
   its stereoisomers, derivatives, solvates and salts thereof.

According to formula (I), n + m is an integer comprised between 1 and 60; nevertheless, in a particular embodiment, n + m is an integer between 1 and lower than 60, e.g., 55 or less, 50 or less, 45 or less, 40 or less, 35 or less, 30 or less, 25 or less, 20 or less, 15 or less, 10 or less, 5 or less. In a particular embodiment n + m is comprised between 1 and 12, preferably, between 2 and 10, most preferably, between 3 and 8.

A particular class of compounds of formula (I) are those compounds of formula (I) in which R is H, hereinafter referred to as compounds of formula (Ia). Illustrative, non-limitative examples of compounds of formula (Ia) include epoxides of terpenes, e.g., epoxides of C₅ hemiterpenes, epoxides of C₁₀ monoterpenes, epoxides of C₁₅ sesquiterpenes, epoxides of C₂₀ diterpenes, epoxides of C₂₅ sesterpenes, epoxides of C₃₀ triterpenes, epoxides of C₄₀ tetraterpenes, etc., for example, epoxides of squalene, etc. In a preferred embodiment, the polyisoprenoid epoxide of the invention is an epoxidated squalene having 1 or 2 epoxide moieties which is a potent inhibitor of cholesterol synthesis (see Table 2 below).

Another particular class of compounds of formula (I) are those compounds of formula (I) in which R is OH, hereinafter referred to as compounds of formula (Ib). Illustrative, non-limitative examples of compounds of formula (Ib) include epoxides of polyprenols, e.g., epoxides of geraniol, epoxides of farnesol, epoxides of geranylgeraniol, epoxides of solanesol, etc. The number of epoxide moieties within said polyisoprenoid epoxides will typically depend, among other things, on the number of double bonds which are present within the starting polyisoprenoid. Thus, the famesol epoxide may contain 1, 2 or 3 epoxide moieties, typically 1 or 2; the geranylgeraniol epoxide may contain 1, 2, 3 or 4 epoxide moieties; typically 1, 2 or 3; the solanesol epoxide may contain up to 9 epoxide moieties, typically 1, 2, 3, 4, 5, 6, 7, 8 or 9, more typically 4 or less. In a preferred embodiment, the polyisoprenoid epoxide of the invention is an epoxidated solanesol containing 1 to 4 epoxide moieties, preferably 3 or 4 epoxide moieties, since said compounds increase CoQ synthesis while practically eliminating cholesterol synthesis (see Table 2 below).

Another particular class of compounds of formula (I) are those compounds of formula (I) in which R is R¹OC(O)-, wherein R¹ is that previously defined, hereinafter referred to as compounds of formula (Ic). Illustrative, non-limitative examples of compounds of formula (Ic) include epoxides of derivatives of polyprenols, e.g., the acetylated, phosphorylated or phtalimidated forms of epoxides of solanesol, etc.

Another particular class of compounds of formula (I) are those compounds of formula (I) in which R is R¹OP(=O)₂O-, wherein R¹ is that previously defined, hereinafter referred to as compounds of formula (Id). Illustrative, non-limitative examples of compounds of formula (Ia) include epoxides of derivatives of polyprenols, etc.

Another particular class of compounds of formula (I) are those compounds of formula (I) in which R is radical (A), wherein R², R³ and R⁴ are those previously defined, hereinafter referred to as compounds of formula (Ie). Radical (A) has been represented in an oxidized state; nevertheless, it may also exist in the reduced state wherein the carbonyl groups of the aromatic ring are reduced to hydroxy groups. In a particular embodiment, the compound of formula (I) is a compound of formula (Ie) wherein R², R³ and R⁴ are methyl and n + m comprises a value from 1 to 10, wherein n is an integer from 0 to 9 and m is an integer from 1 to 10; preferably m is an integer between 1 to 6, preferably from 2 to 5 and even more preferably 3 or 4. Illustrative, non-limitative examples of compounds of formula (Ie) include epoxides of CoQ, etc. In a preferred embodiment, the polyisoprenoid epoxide of the invention is an epoxidated derivative of CoQ containing 1 to 4 epoxide moieties, preferably epoxidated derivatives of CoQ containing 3 or 4 epoxide moieties (Figure 2B).

Another particular class of compounds of formula (I) are those compounds of formula (I) in which R is radical (B), wherein R⁴, R⁵, R⁶, R⁷, R⁸ and q are those previously defined, hereinafter referred to as compounds of formula (If). In a particular embodiment, the compound of formula (I) is:
- a compound of formula (If) wherein R⁵ is OH, and R⁴, R⁶, R⁷ and R⁸ are methyl;
- a compound of formula (If) wherein R⁵ is OH, R⁴, R⁷ and R⁸ are methyl, and R⁶ is H;
- a compound of formula (If) wherein R⁵ is OH, R⁴ is H, and R⁶, R⁷ and R⁸ are methyl; or
- a compound of formula (If) wherein R⁵ is OH, R⁴ and R⁶ are both H, R⁷ and
R⁸ are both methyl.

Illustrative, non-limitative examples of compounds of formula (If) include epoxides of tocotrienols (e.g., α, β, γ, δ). In a preferred embodiment, the polyisoprenoid epoxide of the invention is an epoxidated tocotrienol containing 1 to 3 epoxide moieties, preferably 1 or, most preferably 2 epoxide moieties. In particular, the epoxidated derivative of δ-tocotrienol having one epoxide moiety and all four α-, β-, γ-, δ-tocotrienols having two epoxide moieties strongly inhibited cholesterol synthesis (Figure 3).

Another particular class of compounds of formula (I) are those compounds of formula (I) in which R is radical (C), wherein R⁴, R⁵, R⁶, R⁷, R⁹ and q are those previously defined, hereinafter referred to as compounds of formula (Ig). Illustrative, non-limitative examples of compounds of formula (Ig) include epoxides of vitamin K₂, etc. In a preferred embodiment, the polyisoprenoid epoxide of the invention is an epoxidated vitamin K₂ since it inhibits cholesterol synthesis and increases CoQ synthesis (Table 2).

Compounds of formula (Ia), (Ib), (Ic) and (Id) are regarded, within the context of the instant invention, as "free polyisoprenoid epoxides", i.e., to distinguish them from the compounds of formula (Ie), (If) and (Ig) which are regarded as "bound polyisoprenoid epoxides", which are bound to an aromatic system.

In a preferred embodiment, the polyisoprenoid epoxide of the invention is selected from the group consisting of:
an epoxidated squalene containing 1 or 2 epoxide moieties;
an epoxidated solanesol containing 1 to 4 epoxide moieties, preferably 3 or 4 epoxide moieties, or a derivative thereof;
an epoxidated derivative of CoQ containing 1 to 4 epoxide moieties, preferably 3 or 4 epoxide moieties;
an epoxidated tocotrienol containing 1 or 2 epoxide moieties;
an epoxidated derivative of δ-tocotrienol having one epoxide moiety;
an epoxidated α-tocotrienol having 2 epoxide moieties;
an epoxidated β-tocotrienol having 2 epoxide moieties;
an epoxidated γ-tocotrienol having 2 epoxide moieties;
an epoxidated δ-tocotrienol having 2 epoxide moieties; and
an epoxidated vitamin K₂.

The skilled person in the art will note that the compounds of formula (I) may also include stereoisomers and diastereomers. The single isomers and mixtures of isomers fall within the scope of the present invention.

Unless otherwise stated, the compounds of formula (I) also include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

Further, the compounds of formula (I) may be in crystalline form either as free compounds or as solvates (e.g. hydrates) and it is intended that both forms are within the scope of the present invention. Methods of solvation are generally known within the art.

### Process

In another aspect, the invention relates to a process for obtaining a polyisoprenoid epoxide of the invention, hereinafter referred to as the process of the invention, which comprises the full or partial oxidation of an *all-trans* polyisoprenoid. If desired, the compounds so obtained can be subjected to additional steps of isolation and purification.

It will be immediately apparent to the skilled person in the art that a wide range of polyisoprenoids epoxide of the invention are accessible from polysoprenoids depending on the oxidation reaction conditions. These reactions have been extensively studied in the art, for example, see pages 1051 to 1054 of "Advanced Organic Chemistry. Reactions, Mechanisms, and Structure" March, J.; Smith, M. B., fifth edition, 2001, Wiley Interscience; also "Essentials of Organic Chemistry" Dewick PM ed., 2006, John Wiley & Sons; and also a recent review on selective epoxidation methods, Rose E. et al, Chem. Soc. Rev. 34, 573-583, 2005.

According to the number (n) of repeating isoprene units [-(CH₂-C(CH₃)=CH-CH₂)- or isoprenyl group] the polyisoprenoid, the starting polyisoprenoid can be short (n = 2 to 8), medium (n = 9 to 24) or long (n larger than 24) and the number of double bonds can vary with the proviso that all double bonds are of trans geometry; further the starting all-*trans* polyisoprenoid may comprise alpha saturated or unsaturated forms and/or omega saturated or unsaturated forms.

All-trans polyisoprenoids, in general, are known or can be obtained by the skilled person in the art by conventional means. Illustrative, non-limitative, examples of *all-trans* polyisoprenoids which can be used as starting materials according to the invention include polyprenols such as farnesol, geraniol, geranylgeraniol, solanesol, etc., terpenes such as squalene, etc., or polyisoprenoids bound to an aromatic system, such as such as CoQ, α-, β-, γ-, or δ-tocotrienols, vitamin K, etc.

According to a particular embodiment, the process of the invention is carried out by an enzyme system. In a more preferred embodiment, the enzyme system contains cytochrome P-450.

Alternatively, according to another particular embodiment, the process of the invention is carried out by subjecting the polyisoprenoid to ultraviolet irradiation under suitable conditions. The election of the most suitable conditions requires only routine experimentation for the skilled person in the art.

Further, according to another particular embodiment, the process of the invention is carried out by using chemical oxidising agents. Illustrative, non-limitative, examples of chemical oxidising agents include peracids, oxygen or alkyl peroxides in the presence of transition metals or magnesium monoperoxyphthalate. The election of the most suitable chemical oxidising agent requires only routine experimentation for the skilled person in the art. When peracids are used, the reaction mixture can be optionally buffered, according to methods known to the skilled person. Examples of organic peracids include, but are not limited to, 3-chloroperoxybenzoic acid, peracetic acid and peroxybenzoic acid.

Also, the election of the most suitable reaction conditions is a matter of routine experimentation for the skilled person in the art and fine tuning of these dictate the reaction outcome. In general, when the polyisoprenoid epoxide is obtained by chemical oxidation, the amount of the oxidizing agent plays an important role. Since, normally, at the end of the oxidation reaction a mixture of different polyisoprenoids epoxides is obtained, it has been observed that by increasing the amount of oxidizing agent, the number of epoxides moieties within the starting polyisoprenoids is increased. The molar ratio "isoprenoid:oxidizing agent" can vary in a broad range, for example, from 10:1 to 1:10. In a particular embodiment, the molar ratio "isoprenoid:oxidizing agent" is comprised between 2:1 and 1:3. In another particular embodiment, it has been found that when the isoprenoid is CoQ or solanesol, a molar ratio "isoprenoid:oxidizing agent" of 2:1, led to the highly selective formation of the mono-epoxidated polyisoprenoid.

Further, if desired, the polyisoprenoid epoxide of the invention, which is a partially or fully oxidised compound obtained according to the process of the invention, can be optionally isolated (separated) and, if desired, purified by conventional techniques known in the art, such as chromatography, precipitation, crystallisation, etc., although the invention also contemplates the use of a mixture comprising twoor more different polyisoprenoids epoxides of the invention.

### Uses

The polyisoprenoid epoxides of the invention influence the biosynthesis of the mevalonate pathway lipids; thus, they can decrease the cholesterol biosynthesis and/or increase the CoQ biosynthesis. Consequently, the polytisoprenoid epoxides of the invention can be used for therapeutical purposes.

Since both high level of blood cholesterol and low blood and tissue levels of CoQ contents are major factors in the development of a number of diseases, the polyisoprenoid epoxides of the invention may be useful as effective therapeutic agents in the treatment of several diseases such as hypercholesterolemia, cardiomyopathy, degenerative muscle diseases, cancer, neurological disorders (e.g., Parkinson's disease, Huntington's disease, Friedreich's ataxia, amyotrophic lateral sclerosis, migraine headache, etc.), bronchial asthma and genetic disorders causing a decreased synthesis of CoQ.

It is, therefore, another aspect of the invention, to provide a polyisoprenoid epoxide of the invention for use as a medicament for treating a disease related to high cholesterol levels, and/or a CoQ deficiency. In a particular embodiment, the polyisoprenoid epoxide of the invention can be used for treating hypercholesterolemia and/or diseases related to a coenzyme Q deficiency such as cardiomyopathy, degenerative muscle diseases, cancer, neurological disorders (e.g., Parkinson's disease, Huntington's disease, Friedreich's ataxia, amyotrophic lateral sclerosis, migraine headache, etc.), bronchial asthma and genetic disorders causing decreased synthesis of CoQ.

Therefore, in another aspect, the invention relates to a pharmaceutical composition, hereinafter referred to as pharmaceutical composition of the invention, comprising a polyisoprenoid epoxide of the invention, or a composition obtainable according to the process of the invention, together with a pharmaceutically acceptable excipient or carrier.

In a particular embodiment, the pharmaceutical composition of the invention comprises, as active ingredient, at least one polyisoprenoid epoxides of the invention, in a therapeutically effective amount.

As mentioned above, the excipients, e.g., pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as excipients, particularly for injectable solutions. Suitable pharmaceutical excipients are described in "Remington's Pharmaceutical Sciences" by E.W. Martin.

For its administration to a subject, such as a mammal, e.g., a human, in need of treatment, the pharmaceutical composition of the invention may be administered by any appropriate route (via), such as, oral (e.g., oral, sublingual, etc.), parenteral (e.g., subcutaneous, intramuscular, intravenous, intramuscular, etc.), vaginal, rectal, nasal, topical, ophtalmic, etc.

The pharmaceutical composition of the invention, obtained by mixing the active ingredient(s) with the suitable pharmaceutically aceptable excipient(s) may be administered in a plurality of pharmaceutical forms of administration, e.g., solid, liquid, etc. Illustrative, non-limitative examples of said pharmaceutical forms of administration of the pharmaceutical composition of the invention include oral drops (solution, suspension, emulsion, etc.); oral formulations (liquid, solution, suspension, emulsion, gel, paste, powder, etc.); powder for oral solution or suspension; granules; gastro-resistant granules; prolonged-release granules; modified-release granules; granules for oral suspension; powder and solvent for oral solution or suspension; syrup; powder for syrup; granules for syrup; tablets (e.g., soluble tablet, dispersible tablet, coated tablet, film-coated tablet, effervescent tablet, orodispersible tablet, gastro-resistant tablet, prolonged-release tablet, modified-release tablet, buccal tablet, chewable tablet, etc.); effervescent powder or granules; sachet, capsule (e.g., hard, soft, gastro-resistant hard or soft capsule, prolonged-release hard or soft capsule, modified-release hard or soft capsule, etc.); premix for medicated feeding staff; pellets, suppository; solution for injection; suspension for injection; emulsion for injection; powder for solution for injection; powder for suspension for injection; powder and solvent for solution for injection; powder and solvent for suspension for injection; concentrate for solution for injection; solution for infusion; emulsion for infusion; powder for solution for infusion; concentrate for solution for infusion; powder and solvent for solution for infusion; etc.

The excipients necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods, excipients to be used and processes for producing them can be found in "Tratado de Farmacia Galénica", C. Faulí i Trillo, Luzán 5, S.A. de Ediciones, 1993.

The polyisoprenoid epoxides of the invention may also be administered with other drugs to provide a combination therapy. The other drugs may form part of the same pharmaceutical composition, or be provided as a separate composition for administration at the same time or at different time.

In another aspect, the invention relates to the use of a polyisoprenoid epoxide of the invention, its stereoisomers, derivatives, solvates or pharmaceutically acceptable salts for the manufacture of a pharmaceutical composition for treating hypercholesterolemia and/or diseases related to a coenzyme Q deficiency, such as cardiomyopathy, degenerative muscle diseases, cancer, neurological disorders (e.g., Parkinson's disease, Huntington's disease, Friedreich's ataxia, amyotrophic lateral sclerosis, migraine headache, etc.), bronchial asthma and genetic disorders causing decreased synthesis of CoQ.

In yet another aspect, the invention relates to a method for the preparation of a pharmaceutical composition for treating hypercholesterolemia and/or a disease related to a CoQ deficiency such as cardiomyopathy, degenerative muscle diseases, cancer, neurological disorders (e.g., Parkinson's disease, Huntington's disease, Friedreich's ataxia, amyotrophic lateral sclerosis, migraine headache, etc.), bronchial asthma and genetic disorders causing decreased synthesis of CoQ, which comprises mixing a polyisoprenoid epoxide of the invention, as active ingredient, with a pharmaceutically acceptable excipient.

The compounds used in the present invention may also be administered with other drugs to provide a combination therapy. The other drugs may form part of the same composition, or be provided as a separate composition for administration at the same time or at different time.

The following examples illustrate the invention. They do not intend or should be interpreted to limit in any way the scope of the invention disclosed in the present description.

### EXAMPLES

### 1. Materials and Methods

### 1.1 Reagents

α-Unsaturated polyprenols were prepared from *Sorbus suecica* as described previously (Chojnacki, T., and Vogtman, T. (1984) Acta Biochim Pol 31(1), 115-126.

Dolichol-11 was prepared by saturation of the α-terminal isoprene residue of plant undecaprenol (Mankowski, T., Jankowski, W., Chojnacki, T., and Franke, P. (1976) Biochemistry 15(10), 2125-2130).

Dolichol-18 was isolated from human liver (Ekstrom, T. J., Chojnacki, T., and Dallner, G. (1984) J Biol Chem 259(16), 10460-10468).

Solanesyl-PP was prepared according to Popjak, G., Cornforth, J. W., Cornforth, R. H., Ryhage, R., and Goodman, D. S. (1962) J Biol Chem 237, 56-61.

The polyprenols were acetylated by treatment with acetic acid anhydride:pyridine (1:1) at room temperature for 1 hour and the resulting products isolated by silica chromatography.

Phtalimidation of solanesol was performed according to Sen, S. E., and Roach, S. L. (1995) Synthesis 1995, 756-758.

Tocotrienols were purchased from Davos Life Sciences Pte Ltd (Singapore). (R,S)-5-[³H]Mevalonolactone was synthesized employing [³H]sodium borohydride (15 Ci/mmol, GE Healthcare) as described by Keller (Keller, R. K. (1986) J Biol Chem 261(26), 12053-12059).

[³H]Lanosterol was synthesized from [³H]mevalonate according to Downs *et al* (Downs, S. M., Ruan, B., and Schroepfer, G. J., Jr. (2001) Biol Reprod 64(1), 80-89).

All other chemicals were procured from Sigma (St Louis).

### 1.2 UV irradiation and separation of products

To obtain compounds formed in response to ultraviolet (UV) irradiation, CoQ₁₀ dissolved in ethanol:water (9:1) (0.4 mg/ml) was irradiated with a UV-lamp at 190-300 nm, 500 µW/cm² for 30 min. Following evaporation of the solvents, the residue was dissolved in hexane and applied to a silica gel column, which was then eluted with a gradient starting from 100% hexane and finishing with hexane:diethyl ether (1:2).

Previously, compounds obtained by subjecting CoQ to UV irradiation and separation by thin layer chromatography (TLC) were analyzed. Briefly, CoQ10 dissolved in ethanol-water 9:1 was irradiated with a UV-lamp (300-400 nm) for 30 min; after irradiation, the reaction mixture was concentrated, the lipids were dissolved in chloroform:methanol (2:1) (v:v) and the whole mixture was analyzed on a TLC plate Silica 60 (Merck), developed with toluene:ethyl acetate:acetic acid (50:5:1) (v:v:v) and visualised with iodine.

### 1.3 Chemical epoxidation

For the purposes of chemical epoxidation, the various lipids were dissolved in dichloromethane and then mixed with 3-chloroperoxybenzoic acid (77%) dissolved in dichloromethane to obtain a 1:2 molar ratio of lipid:3-chloroperoxybenzoic acid (Duitsman, P. K., Barua, A. B., Becker, B., and Olson, J. A. (1999) Int J Vitam Nutr Res 69(5), 303-308). Following incubation at room temperature for 30 min, the solvent was removed by evaporation under nitrogen and the residue re-dissolved in hexane:diethyl ether (4:1). Subsequently, the individual epoxides were separated by column chromatography on silica gel 60 (230-400 mesh) using a gradient from hexane to an equal mixture of hexane and diethyl ether for elution. The individual fractions thus obtained were purified further on a RP-18 column (LiCroprep 40-63 µm, Merck) with methanol:acetone (3:1) as the eluent.

### 1.4 Cell cultures

Human hepatoblastoma (HepG2) cells (cell line derived from a human liver cancer that contains primarily CoQ with a decaprenyl side-chain available in ATCC (Cat. No. HB-8065), obtained from LGC Prochem, UK) were cultured in 10 ml DMEM medium (Invitrogen) containing 1 g glucose per 100 ml, 10% fetal bovine serum, penicillin (100 units per ml) and streptomycin (100 mg/ml). When 70% confluency was reached, the medium was changed and the compounds to be investigated added in 50-100 µl dimethylsulfoxide to give a final concentration of 2 µM or in the case of tocotrienols 5 µM. Twenty-four hours later, 0.5 mCi [³H]mevalonate (3.25 Ci/mmol) was added and incubation continued for an additional 8 hours. When the substrate was [³H]lanosterol (0.28 Ci/mmol) the amount added was 5 µCi. Finally, the cells were harvested by trypsinisation and stored at -20° C for later analysis.

### 1.5 Extraction and chromatography of lipids

Lipids were extracted from cells with chloroform:methanol:water (1:1:0.3), at 37°C for 1 hr with magnetic stirring (Ericsson, J., Runquist, M., Thelin, A., Andersson, M., Chojnacki, T., and Dallner, G. (1993) J Biol Chem 268(2), 832-838). The extracts thus obtained were adjusted to achieve a final chloroform:methanol:water ratio of 3:2:1 and complete phase separation then accomplished by centrifugation. The lower chloroform phase was removed and evaporated to dryness under a flow of nitrogen and the residue subsequently re-dissolved in chloroform. These solutions were placed onto a silica column (50 mg/1.5 ml; Extract-Clean, Alltech, Deerfield, IL) and thereafter eluted with 6 ml chloroform. After evaporation of the solvents, the neutral lipids were dissolved in chloroform:methanol (2:1).

For analysis of cholesterol, cholesterol metabolites, squalene, and CoQ, the samples were subjected to reversed-phase HPLC using a Supelcosil LC-18 column (3 µm, 4.0 x 75 mm) equipped with an LC-18 Supelguard (Supelco) column (Ericsson, J., Thelin, A., Chojnacki, T., and Dallner, G. (1992) J Biol Chem 267(27), 19730-19735). For separation, a stepwise gradient involving methanol:water (9:1) in pump system A and methanol:2-propanol:hexane (2:1:1) in pump system B was employed at a flow rate of 1.1 ml/min. The gradient began at 15% solvent B, with increases to 47% after 8 min, 67% after 16 min and 100% after 24 min. The lipids in the eluent were monitored at 210 nm and 275 nm with a UV detector and labeling of radioactive products was measured with on-line detector of radioactivity. Ergosterol and CoQ₆ were used as internal standards.

For the analysis of individual products following irradiation or chemical epoxidation of CoQ, this same HPLC procedure was utilized. However, HPLC analysis of tocotrienol epoxides was performed with a linear gradient starting from methanol:water at a ratio of 7:3 and ending with methanol:water at a ratio of 9:1. The flow rate was 1.1 ml/min and the program time 25 min.

Following chemical epoxidation, the products were separated by silica gel column chromatography as described above. Subsequently, the individual fractions were analyzed on silica gel-60 plates (Merck) and HPTLC RP-18 plates (Merck) and the spots were visualized with iodine. In the case of CoQ, solanesol, squalene, vitamin K₂ and polyprenols 7 and 11, the solvent system used for development was benzene:ethyl acetate (9:1) for the silica gel-60 plates and acetone:methanol (2:1) for HPTLC RP-18 plates. For farnesol and geranylgeraniol the corresponding solvent systems were, benzene:ethyl acetate (4:1) and acetone:methanol (1:1); while for dolichols, benzene:ethyl acetate (9:1) and 100% acetone were employed respectively. Finally, benzene:ethyl acetate (6:1) and acetone:methanol:water (6:3:1) were used for the separation of tocotrienols.

### 1.6 Mass spectrometry and determination of protein

Negative-ion ES spectra were recorded on a triple quadrupole mass spectrometer as described earlier (Bentinger, M., Dallner, G., Chojnacki, T., and Swiezewska, E. (2003) Free Radic Biol Med 34(5), 563-575). Protein was determined with the bicinchoninic acid kit (Sigma).

### 1.7 Real-time PCR

Quantitative real time PCR was employed to access levels of gene expression. For this purpose, total RNA was isolated from HepG2 cells using the QIAGEN Mini RNeasy plus mini kit and 100 ng of this RNA reverse-transcribed into cDNA using random hexamer oligonucleotides and TaqMan reverse transcription reagents in a final volume of 20 µl. This cDNA was then mixed with SYBR Green PCR master mix and appropriate primers and PCR analysis performed with an ABI Prism 7300 Sequence Detector (Applied Biosystems). The genes analyzed and the respective primers are shown in Table 1.

**Table 1**

| **Primers used for real-time PCR** | | |
|---|---|---|
| **Gene** | **Forward primer*** | **Reverse primer*** |
| Coenzyme Q1 | TGGTCCACTGAAAGACATGC (SEQ ID NO: 1) | TCCACAACCTTGGTGTTCTG (SEQ ID NO: 2) |
| Coenzyme Q2 | CAACTAGCCTTGGGCTTGAC (SEQ ID NO: 3) | ATGGATCACAGGAACCCTTG (SEQ ID NO: 4) |
| Farnesyl-PP synthase | AGGTGGCTGGGTTCCCTAC (SEQ ID NO: 5) | TCTGGTCTCCGTTCATTCTGA (SEQ ID NO: 6) |
| Squalene synthase | CAGACCAGTCGCAGTTTCG (SEQ ID NO: 7) | CAAGGGAGATCGTTGGGAAGT (SEQ ID NO: 8) |
| Cyclophilin B | GGCACAGGAGGAAAGAGCAT (SEQ ID NO: 9) | GCCCGTAGTGCTTCAGTTTG (SEQ ID NO: 10) |

| | | |
|---|---|---|
| *5' to 3' | | |

### 1.8 Statistical analyses

The results obtained are expressed as means ± SD and the differences between two groups evaluated for statistical significance by Student's *t*-test. Differences between several groups were analyzed by ANOVA, followed by Dunnett's test. P values below 0.05 were considered statistically significant.

### 2. Results

### 2.1 Effects of UV light on CoQ₁₀

Irradiation of a solution of CoQ₁₀ and subsequent evaporation of the solvent and separation by thin layer chromatography (TLC) revealed a large number of products which were more hydrophilic than the parent lipid, as well as some more hydrophobic compounds. Consequently, in order to obtain fractions for a more detailed analysis, the irradiated mixture was subjected to column chromatography on silica gel employing elution with a solvent mixture of increasing hydrophilicity. When aliquots of the fractions were added to cultures of HepG2 cells (a cell line derived from a human liver cancer that contains primarily CoQ with a decaprenyl side-chain), six of the fractions obtained resulted in a reduced rate of incorporation of [³H]mevalonate into cholesterol (Figure 1A). At the same time, CoQ synthesis was enhanced to various extents (Figure 1B). As illustrated for fraction 28 (Figure 1C), fractions collected from the silica gel column were found to contain heterogeneous and complex mixtures of compounds upon analysis by reversed-phase HPLC.

### 2.2 Effects of chemically epoxidated CoQ and solanesol

Certain of the peaks in the HPLC chromatograms exhibited elution times identical to derivatives of CoQ containing 1-4 epoxide moieties (prepared by treating CoQ₁₀ with 3-chloroperoxybenzoic acid) and could be separated efficiently by column chromatography on silica gel, followed by chromatography on C18 HPTLC plates (Figure 2A). Mass spectrometric analysis confirmed that the molecular weights of the compounds thus obtained were those of CoQ₁₀ +16, +32, +48 and +64 Daltons, respectively. Furthermore, although the side-chain contained one or more epoxide moieties, spectroscopic investigation demonstrated that the quinone ring had not been modified.

The biological effects of chemically epoxidated CoQ₁₀ on HepG2 cells were examined. Cholesterol synthesis by HepG2 cells was not influenced by the presence of derivatives of CoQ with 1 or 2 epoxide moieties in the culture medium, but was potently reduced by derivatives of CoQ containing 3 or 4 such moieties (Figure 2B). In contrast, biosynthesis of CoQ was enhanced by all four types of derivatives. Since these analyses showed that only the polyisoprenoid side-chain is modified in these derivatives, the effects of the corresponding epoxides of solanesol, the side-chain of CoQ₉, were also tested. Solanesol containing 1-4 epoxide moieties elevated CoQ synthesis while practically eliminating cholesterol synthesis.

### Effects of epoxidated derivatives of various poly-cis and all-trans polyisoprenoids

Poly-*cis* α-unsaturated polyprenols constitute the majority of isoprenoid lipids that occur naturally, particularly in plants, whereas in animal tissues α-saturated dolichols are predominant. Two α-unsaturated polyprenols (7 and 11) and two α-saturated polyprenols (11 and 18) were epoxidated and these derivatives found to exert only minor effects on the biosynthesis of cholesterol and CoQ by HepG2 cells (Table 2).

**Table 2**

| **Biosynthesis of cholesterol and CoQ by HepG2 cells in the presence of various polyisoprenoid epoxides** | | | |
|---|---|---|---|
| **Polyisoprenoid epoxides** | | **Cholesterol** | **Coenzyme Q₁₀** |
| | | *% of control* | |
| | Control | 100 ± 4 | 100 ± 6 |
| Poly-cis | | | |
| | Polyprenol 7-cis | 92 ± 4 | 114 ± 9 |
| | Polyprenol 11-cis | 92 ± 5 | 106 ± 5 |
| | Dolichol-11 | 93 ± 6 | 113 ± 10 |
| | Dolichol-18 | 96 ± 3 | 114 ± 5 |
| *All-trans* | | | |
| | Farnesol | 101 ± 3 | 100 ± 4 |
| | Farnesyl-acetate | 103 ± 4 | 105 ± 8 |
| | Geranylgeraniol | 102 ± 5 | 107 ± 11 |
| | Geranylgeranyl-acetate | 102 ± 4 | 109 ± 12 |
| | Vitamin K₂ | 67 ± 7 **^{***}** | 127 ± 18 ^{*} |
| | Squalene | 30 ± 5 ^{***} | 111 ± 8 |
| | Solanesol | 3 ± 1 ^{***} | 138 ± 14 ^{***} |
| | Solanesyl-phospate | 7 ± 2 ^{***} | 136 ± 13 ^{***} |
| | Solanesyl-acetate | 4 ± 2 ^{***} | 145 ± 18 ^{***} |
| | Solanesyl-phtalimide | 3 ± 1 ^{***} | 142 ± 17 ^{***} |

| | | | |
|---|---|---|---|
| Various polyprenols, their derivatives, vitamin K₂ and squalene were epoxidated and the resulting compounds purified by chromatography on silica gel. The poly-cis compounds contained 1-4 epoxide moieties, farnesol and farnesyl-acetate and squalene 1 and 2 epoxide moieties, geranylgeraniol and its acetate form and vitamin K₂ 1-3 epoxide moieties, and solanesol and its derivatives 1-4 epoxide moieties. These compounds were added to the medium of HepG2 cells at a final concentration of 2 µM and 24 hours later this medium was supplemented with [³H]mevalonate, followed by an additional 8 hours of incubation, extraction of cellular lipids and separation by HPLC with on-line detection of radioactivity in order to determine the extent of labeling of cholesterol and CoQ. The values presented are means ± SD (n = 4). **P*<0.05, ****P*<0.001. | | | |

Epoxidated derivatives of the two short all-*trans* lipids farnesol and geranylgeraniol (which were also acetylated in order to enhance their uptake into the cells) did not alter cholesterol or CoQ synthesis significantly.

Epoxidated vitamin K₂ inhibited cholesterol synthesis by 33% and elevated CoQ synthesis by 27%.

Epoxidated squalene (possessing one or two epoxide moieties in the middle portion of the isoprenoid chain, in contrast to the natural substrate for cholesterol biosynthesis 2,3-oxidosqualene) proved to be a potent inhibitor of cholesterol synthesis (70% reduction).

Epoxidated derivatives of solanesol and its phosphorylated, acetylated and phtalimidated forms produced virtually total inhibition of cholesterol synthesis and, at the same time, stimulated CoQ synthesis by approximately 40%.

All of the epoxidated isoprenoids were tested in a concentration of 2 µM. At this concentration none of the parent non-epoxidated compounds exerted any effects on cholesterol or CoQ synthesis (not shown).

### Effects of epoxy tocotrienols

Tocopherols contain a phytol-type side-chain, whereas the side-chain of tocotrienols consists of three unsaturated isoprenoid residues. Four types of tocotrienols, with differing numbers and distributions of methyl groups on the chromanol ring, occur naturally (α, β, γ, and δ-tocotrienols). Employing the same procedure for epoxidation as described above, tocotrienols possessing one or two epoxide moieties were produced and subsequently isolated by chromatography.

The rate of cholesterol biosynthesis by HepG2 cells was unaffected by the presence of the four non-derivatized tocotrienols themselves (Figure 3A). Similarly, α, β and γ-tocotrienols with one epoxide moiety were without effect on this rate. In sharp contrast, the δ-form with one epoxide moiety and all four tocotrienols (α, β, γ, and δ-tocotrienols) with two epoxide moieties inhibited cholesterol synthesis potently, decreasing labeling by 56-93%.

These tocotrienol epoxides exerted opposite effects on the biosynthesis of CoQ (Figure 3B). To various and moderate extents, the parent compounds enhanced the rate of this process; whereas all of the derivatives containing one or two epoxide moieties stimulated CoQ biosynthesis 2.5-3.5-fold. Thus, certain of the tocotrienol epoxides selectively elevated CoQ synthesis, while others also reduced the rate of cholesterol biosynthesis by HepG2 cells.

In order to investigate the toxicity of tocotrienol epoxides in the cultures, HepG2 cells were cultured in the presence of various tocotrienol epoxides at concentrations (60 µM) 12-fold higher than those routinely employed here. The morphology, growth and lipid metabolism of the cells were not altered under these conditions, indicating a very low degree of toxicity.

### Effects of prolonged treatment

In the experiments described above, rates of biosynthesis were determined by monitoring incorporation of [³H]mevalonate. The potential physiological impact is, however, dependent on the changes in the actual amounts of CoQ. Therefore, HepG2 cells were cultured for 26 days in the presence of monoepoxy-γ-tocotrienol (which stimulates CoQ biosynthesis), during which period the cellular lipid composition was analyzed (Figure 4). The cellular content of CoQ increased continuously, reaching a level after 26 days of treatment that was 30% higher than in untreated, control cultures. At the same time the cellular content of cholesterol was not altered, as expected, since γ-tocotrienol with only one epoxide moiety does not influence the biosynthesis of this steroid by HepG2 cells.

### Effects on mRNA levels

In order to elucidate the mechanism underlying the effects of these various epoxide derivatives on CoQ biosynthesis by HepG2 cells, the levels of mRNA encoding the key enzymes involved in this process were determined by real-time PCR during treatment with monoepoxy-γ-tocotrienol (Table 3). Already after 15 minutes of such treatment, the levels of mRNA species coding for COQ1 and COQ2, the enzymes that catalyze the first two steps in this process (one of which is considered to be rate-limiting) were elevated significantly. At the same time, the levels of mRNA for famesyl-PP synthase and squalene synthase were not altered.

**Table 3**

| **Levels of mRNA expression COQ1, COQ2, FDPS and SQS in HepG2 cells incubated together with monoepoxy-**γ**-tocotrienol** | |
|---|---|
| | **Change** % of control |
| COQ1 | 228 ± 42 ^{***} |
| COQ2 | 156 ± 26 ^{*} |
| FDPS | 106 ± 15 |
| SQS | 110 ± 14 |

| | |
|---|---|
| HepG2 cells were cultured in the presence of 5 µM monoepoxy-γ-tocotrienol for 15 min, then total RNA was isolated and the individual mRNA species quantified by real-time PCR as described in the Materials and Methods. COQ1 encodes the enzyme *trans*-prenyltransferase, COQ2 encodes decaprenyl-4-hydroxybensoate transferase, FDPS encodes farnesyl-PP synthase and SQS encodes squalene synthase. The values given are means ± SD (n = 4). **P*<0.05, ****P*<0.001. | |

### The site of inhibition of cholesterol synthesis

When HepG2 cells were incubated with [³H]mevalonate alone, radioactivity was recovered in cholesterol and in squalene, the first intermediate after the branch point (Figure 5A). When the solanesol derivative containing two epoxide moieties was also present in the culture medium, labeling of cholesterol was greatly reduced and in addition new radioactive peaks appeared in the HPLC choromatogram (Figure 5B). Through the use of standards and mass spectrometric analysis, these peaks were identified as 2,3-oxidosqualene which is the substrate of oxidosqualene cyclase, as well as 2,3:22,23-dioxidosqualene and 24(S),25-epoxycholesterol which are formed when squalene is epoxidated at both ends. Previous studies have demostrated that these intermediates accumulate when oxidosqualene cyclase is inhibited (Cattel, L., Ceruti, M., Balliano, G., Viola, F., Grosa, G., Rocco, F., and Brusa, P. (1995) Lipids 30(3), 235-246; Mark, M., Muller, P., Maier, R., and Eisele, B. (1996) J Lipid Res 37(1), 148-158).

To further analyze the site of inhibition, RO 48-8071 [(4-bromophenyl)[2-fluoro-4-[[(6-(methyl-2-propenylamino)hexyl]oxy]phenyl]-methanone], an inhibitor of oxidosqualene cyclase and ketoconazole, which is an inhibitor of the next enzyme in the cholesterol synthesis, lanosterol demethylase, was employed (Morand, O. H., Aebi, J. D., Dehmlow, H., Ji, Y. H., Gains, N., Lengsfeld, H., and Himber, J. (1997) J Lipid Res 38(2), 373-390; Trzaskos, J. M., Fischer, R. T., and Favata, M. F. (1986) J Biol Chem 261(36), 16937-16942) (Table 4).

**Table 4**

| S**ynthesis of cholesterol and coenzyme Q in the presence of RO 48-8071 and ketoconazole** | | | | | |
|---|---|---|---|---|---|
| **Inhibitor** | **Cholesterol** | **2,3-Oxido squalene** | **2,3:22,23-Dioxidosqualene** | **Lanosterol** | **CQ₁₀** |
| | *cpm*/*pg protein* | | | | |
| None | 191 ± 12 | n.d. | n.d. | n.d. | 2.3 ± 0.2 |
| Tetraepoxysolanesol | n.d. | 82 ± 8 | 114 ± 25 | n.d. | 3.0 ± 0.4 |
| RO 48-8071 | n.d. | 91 ± 10 | 108 ± 22 | n.d. | 2.1 ± 0.2 |
| Ketoconazole | 25 ± 4^{***} | n.d. | n.d. | 58 ± 16 | 2.4 ± 0.3 |

| | | | | | |
|---|---|---|---|---|---|
| n.d., not detectable Culturing of HepG2 cells was performed in the presence of 2 µM tetraepoxysolanesol or ketoconazole or 0.2 µM RO 48-8071 for 24 hrs followed by [³H]mevalonate for an additional 8 hrs. The lipids were extracted and analyzed on HPLC using on-line detection of radioactivity. The values are means ± SE of 4 independent experiments. ****P*<0.001. | | | | | |

Tetraepoxysolanesol was used in this experiment since this epoxidated isoprenoid is an efficient inhibitor of cholesterol synthesis. Incubation of the cells with this compound eliminated cholesterol labeling and resulted in the accumulation of 2,3-oxidosqualene and 2,3:22,23-dioxidosqualene. Upon incubation with the oxidosqualene cyclase inhibitor RO 48-8071 the pattern was very similar as 2,3-oxidosqualene and 2,3:22,23-dioxidosqualene were accumulated. Ketoconazole in the medium gave a different result, leading to the accumulation of lanosterol.

When [³H]lanosterol was used as precursor, neither tetraepoxysolanesol or RO 48-8071 inhibited the cholesterol synthesis (Table 5).

**Table 5**

| **Biosynthesis of cholesterol from [H³]lanosterol in the presence of inhibitors** | |
|---|---|
| **Inhibitor** | **Cholesterol** |
| | *cpm*/*pg protein* |
| None | 10.5 ± 0.8 |
| Tetraepoxysolanesol | 12.7 ± 1.5 |
| RO 48-8071 | 10.1 ± 1.2 |
| Ketoconazole | 1.3 ± 0.3^{***} |

| | |
|---|---|
| HepG2 cells were cultured in the presence of of 2 µM tetraepoxysolanesol or ketoconazole or 0.2 µM RO 48-8071 for 24 hrs followed by [³H]lanosterol for an additional 8 hrs. The lipids were extracted and analyzed on HPLC. The values are means ± SE of 3 independent experiments. ****P*<0.001. | |

Contrary, ketoconazole inhibited labeling of this lipid. Thus, the epoxidated isoprenoids inhibit cholesterol synthesis at the level of oxidosqualene cyclase and do not influence the next enzyme in the biosynthesis, which is lanosterol demethylase.

Additionally, the experiments in Table 3 also demonstrate that the increase in CoQ labeling observed with various isoprenoid epoxides is not the result of increased availability of FPP to trans-prenyltrasferase since both inhibitors RO 48-8071 and ketoconazole inhibit cholesterol synthesis without elevating CoQ labeling.

When the effects of solanesol derivatives with 1-4 epoxide moieties on HepG2 cells were compared, the solanesol derivative with a single such moiety was found to reduce the incorporation of [³H]mevalonate into cholesterol by 50%, while with the solanesol derivative with 2 epoxide moieties, cholesterol synthesis was decreased by 90%. (Figure 5C). With solanesol derivative with three epoxide moieties, cholesterol biosynthesis was eliminated and labeled 2,3-oxidosqualene, 2,3:22,23-dioxidosqualene and 24(S),25-epoxycholesterol were detected in the cells. Finally, the solanesol derivative containing four epoxide moieties completely eliminated production of both cholesterol and 24(S),25-epoxycholesterol, allowing only 2,3-oxidosqualene and 2,3:22,23-dioxidosqualene to accumulate.

The epoxidation process employed here introduces epoxide moieties at various positions in solanesol simultaneously and thus can be expected to produce a large number of different products with different distributions of epoxidated double bonds. When the diepoxysolanesol fraction after silica chromatography is analyzed on HPLC, several compounds with somewhat variable retention time appear (Figure 5D). It is possible that not only the number, but also the exact location of the epoxide moieties in such compounds may play a role in their biological actions.

### 3. Discussion

The results show that while epoxidated derivatives of short *all-trans* polyprenols, i.e., famesol and geranylgeraniol, exert little influence on lipid metabolism in HepG2 cells, other epoxidated derivatives of other compounds (e.g., vitamin K₂, squalene and solanesol) exerted a great influence on cholesterol and CoQ biosynthesis. Further, tocotrienols with an epoxidated side-chain exert marked effects on cholesterol and CoQ biosynthesis. These effects are highly dependent on the number of epoxide moieties present.

Although it has also been proposed that γ- and δ-tocotrienols can attenuate cholesterol biosynthesis by stimulating the ubiquitination and consequent degradation of HMG-CoA reductase, as well as by blocking the processing of proteins that bind to sterol-responsive elements in the promoters of genes (Song, B. L., and DeBose-Boyd, R. A. (2006) J Biol Chem 281(35), 25054-25061), under the experimental conditions assayed, the synthesis of cholesterol by HepG2 cells was not influenced by tocotrienols. In contrast, tocotrienol epoxides exerted profound effects on the synthesis of both CoQ and cholesterol. Tocotrienols are present in palm and rice oils, from which they can be purified in large quantities and stable form. Subsequent chemical epoxidation is highly efficient and the resulting derivatives containing one or two epoxide moieties can be prepared with high purity. Even high concentrations of such epoxides are non-toxic towards cell cultures. Therefore, if polyisoprenoid epoxides influence CoQ synthesis in humans in a manner similar to that observed in cultures of HepG2 cells, these compounds may prove valuable for treating the CoQ deficiency associated with a number of diseases. In on-going investigations carried out by the inventors on fibroblasts obtained from children carrying genetic defects in CoQ biosynthesis, tocotrienol epoxides have been found to induce the biosynthesis and increase the content of CoQ, indicating a possible strategy for treatment.

The results show that some of the polyisoprenoid epoxides tested here are effective inhibitors of oxidosqualene cyclase, which catalyzes the formation of lanosterol. Thus, said compounds are of potential value for inhibiting the terminal reactions involved in cholesterol synthesis without inhibiting the synthesis of other mevalonate pathway lipids. Moreover, the tocotrienol derivatives containing two epoxide moieties both stimulate CoQ biosynthesis and inhibit cholesterol synthesis, may be of special interest. In addition, inhibition of oxidosqualene cyclase leads to accumulation of 24(S),25-epoxycholesterol, which inhibits cholesterol synthesis by several mechanisms and thereby contributes to the overall inhibitory effect.

The mechanism of action of polyisoprenoid epoxides may be explained by their effects on the biosynthesis of the mevalonate pathway. This pathway has an established sequence of enzymatic reactions, such as those shown in Figure 6. Polyisoprenoid epoxides of the invention inhibit one of the enzymes at the terminal part of cholesterol biosynthesis and thereby decrease the production of end product cholesterol.

## Claims

1. A polyisoprenoid epoxide of an all-*trans* polyisoprenoid or a derivative thereof, which comprises:
a) at least, 2 isoprene units
b) at least, 1 epoxide moiety;
c) at least, 1 double bond; and
wherein the conformation of all the double bonds which are present in said polyisoprenoid epoxide is *trans.*

2. Polyisoprenoid according to claim 1, which comprises from 2 to 100 isoprene units, from 1 to 100 epoxide moieties, from 1 to 100 double bonds; and wherein the conformation of all the double bonds which are present in said compound is *trans.*

3. Polyisoprenoid epoxide according to claim 1 or 2, further comprising a saturated or unsaturated alpha end and/or a saturated or unsaturated alpha end.

4. Polyisoprenoid epoxide according to claim 1 or 2, having formula (I)
R-(X)ₙ-(Y)ₘ-Z (I)
wherein 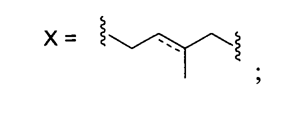 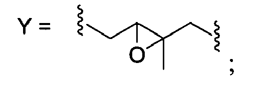 X and Y and can be randomly alternated; 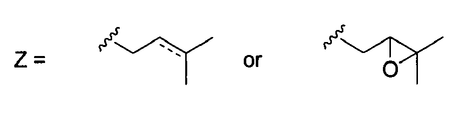 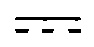 represents an optionally saturated or unsaturated carbon-carbon bond;
n + m is comprised between 1 and 60, wherein n is an integer from 0 to 59 and m is an integer from 1 to 60, and the repeated units can be independently saturated or unsaturated; and
R is selected from the group consisting of H, OH, R¹OC(O)-, R¹OP(=O)₂O-, a radical of formula (A), a radical of formula (B) or a radical of formula (C) 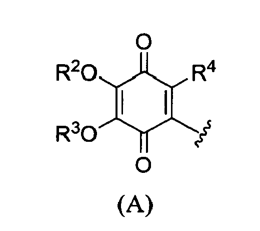 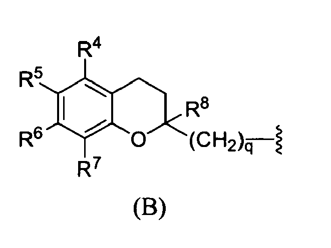 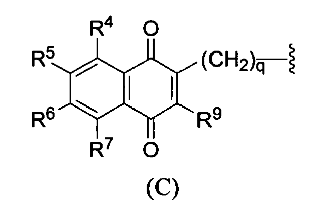 wherein
R¹, R² and R³, independently, are selected from the group consisting of hydrogen, alkyl, cycloalkyl, alkenyl, alkynyl, aryl, aryl-hydrogenated and heterocyclyl; and, optionally, R² and R³ together form (i) a straight or branched divalent radical consisting only of carbon and hydrogen atoms containing no unsaturation and having from 2 to 10 carbon atoms; or (ii) a straight or branched unsaturated divalent radical consisting only of carbon and hydrogen atoms having one or more double bonds and/or one or more triple bonds and having from 2 to 10 carbon atoms, wherein said double or triple bonds can be unconjugated or conjugated to another double or triple bonds unsaturated group, said divalent radicals (i) and (ii) being optionally substituted with one or more substituents selected from halogen, hydroxy, acyl, alkoxy, alkoxycarbonyl, aryloxy, aryloxycarbonyl, carbonyl, carboxy, cyano, amino, imino, nitro, mercapto or alkylthio;
R⁴, R⁵, R⁶, R⁷ and R⁸, independently, are selected from the group consisting of hydrogen, hydroxyl, alkyl, cycloalkyl, alkenyl, alkynyl, aryl, aryl-hydrogenated, heterocyclyl, cyano, -COR¹⁰, - C(O)OR¹⁰, -C(O)NR¹⁰R¹¹, -C=NR¹⁰, -OR¹⁰, OC(O)R¹⁰, -S(O)ₚR¹⁰, -NR¹⁰R¹¹, -NR¹⁰C(O)R¹¹, -NO₂, -N=CR¹⁰R¹¹ or halogen; and, optionally, R⁵ and R⁶ together form (i) a straight or branched divalent radical consisting only of carbon and hydrogen atoms containing no unsaturation and having from 2 to 10 carbon atoms; or (ii) a straight or branched unsaturated divalent radical consisting only of carbon and hydrogen atoms having one or more double bonds and/or one or more triple bonds and having from 2 to 10 carbon atoms, wherein said double or triple bonds can be unconjugated or conjugated to another double or triple bonds unsaturated group, said divalent radicals (i) and (ii) being optionally substituted with one or more substituents selected from halogen, hydroxy, acyl, alkoxy, alkoxycarbonyl, aryloxy, aryloxycarbonyl, carbonyl, carboxy, cyano, amino, imino, nitro, mercapto or alkylthio; wherein R¹⁰ and R¹¹, independently, are selected from hydrogen, alkyl, cycloalkyl, alkenyl, alkinyl, aryl, aryl-hydrogenated, heterocyclyl, alkoxy or aryloxy;
R⁹ is hydrogen or alkyl;
p can take the value 0, 1 or 2; and
q is 0 or 1;
its stereoisomers, derivatives, solvates and salts thereof.

5. Polyisoprenoid epoxide according to claim 1, selected from the group consisting of:
an epoxidated squalene containing 1 or 2 epoxide moieties;
an epoxidated solanesol containing 1 to 4 epoxide moieties, preferably 3 or 4 epoxide moieties, or a derivative thereof;
an epoxidated derivative of CoQ containing 1 to 4 epoxide moieties, preferably 3 or 4 epoxide moieties;
an epoxidated tocotrienol containing 1 or 2 epoxide moieties;
an epoxidated derivative of δ-tocotrienol having one epoxide moiety;
an epoxidated α-tocotrienol having 2 epoxide moieties;
an epoxidated β-tocotrienol having 2 epoxide moieties;
an epoxidated γ-tocotrienol having 2 epoxide moieties;
an epoxidated δ-tocotrienol having 2 epoxide moieties; and
an epoxidated vitamin K₂.

6. A process for obtaining a polyisoprenoid epoxide according to any one of claims 1 to 5, which comprises the full or partial oxidation of an all-*trans* polyisoprenoid, followed, if desired, by isolation and purification.

7. Process according to claim 6, wherein the oxidation of the all-*trans* polyisoprenoid is carried out by an enzyme system; by ultraviolet irradiation; or by using a chemical oxidising agent.

8. A pharmaceutical composition comprising a polyisoprenoid epoxide according to any one of claims 1 to 5, or a steroisomer, derivative, solvate or pharmaceutically acceptable salt thereof, together with a pharmaceutically acceptable excipient or carrier.

9. A polyisoprenoid epoxide according to any one of claims 1 to 5, or a steroisomer, derivative, solvate or pharmaceutically acceptable salt thereof, for treating a disease related to high cholesterol levels, and/or a CoQ deficiency.

10. Polyisoprenoid epoxide according to claim 9, for treating a cardiomyopathy, a degenerative muscle disease, a cancer, a neurological disorder, bronchial asthma or a genetic disorder causing decreased synthesis of CoQ.


SEQUENCE LISTING

<110> Newbiotechnic, S.A.
Universidad Pablo de Olavide
University of Stockholm and Huddinge Hospital

<120> Polyisoperenoid epoxides useful for decreasing cholesterol and/or
increasing coenzyme Q biosynthesis

<130> P2919EP00

<160> 10

<170> PatentIn version 3.3

<210> 1
<211> 20
<212> DNA
<213> Artificial

<220>
<223> Forward primer for analysing, in combination with SEQ ID NO: 2,
the expression of trans-prenyltransferase (CoQ1) by RT-PCR

<400> 1
tggtccactg aaagacatgc 20

<210> 2
<211> 20
<212> DNA
<213> Artificial

<220>
<223> Reverse primer for analysing, in combination with SEQ ID NO: 1,
the expression of trans-prenyltransferase (CoQ1) by RT-PCR

<400> 2
tccacaacct tggtgttctg 20

<210> 3
<211> 20
<212> DNA
<213> Artificial

<220>
<223> Forward primer for analysing, in combination with SEQ ID NO: 4,
the expression of decaprenyl-4-hydroxybenzoate transferase (CoQ2)
by RT-PCR

<400> 3
caactagcct tgggcttgac 20

<210> 4
<211> 20
<212> DNA
<213> Artificial

<220>
<223>  Reverse primer for analysing, in combination with SEQ ID NO: 3,
       the expression of decaprenyl-4-hydroxybenzoate transferase (CoQ2)
       by RT-PCR

<400>  4
atggatcaca ggaacccttg                                                  20


<210>  5
<211>  19
<212>  DNA
<213>  Artificial

<220>
<223>  Forward primer for analysing, in combination with SEQ ID NO: 6,
       the expression of farnesyl-PP synthase (FDPS) by RT-PCR

<400>  5
aggtggctgg gttccctac                                                   19


<210>  6
<211>  21
<212>  DNA
<213>  Artificial

<220>
<223>  Reverse primer for analysing, in combination with SEQ ID NO: 5,
       the expression of farnesyl-PP synthase (FDPS) by RT-PCR

<400>  6
tctggtctcc gttcattctg a                                                21


<210>  7
<211>  19
<212>  DNA
<213>  Artificial

<220>
<223>  Forward primer for analysing, in combination with SEQ ID NO: 8,
       the expression of squalene synthase by RT-PCR

<400>  7
cagaccagtc gcagtttcg                                                   19


<210>  8
<211>  21
<212>  DNA
<213>  Artificial

<220>
<223>  Reverse primer for analysing, in combination with SEQ ID NO: 7,
       the expression of squalene synthase by RT-PCR

<400>  8
caagggagat cgttgggaag t                                                21

<210>  9
<211>  20
<212>  DNA
<213>  Artificial

<220>
<223>  Forward primer for analysing, in combination with SEQ ID NO: 10,
       the expression of cyclophilin B by RT-PCR

<400>  9
ggcacaggag gaaagagcat                                                    20


<210>  10
<211>  20
<212>  DNA
<213>  Artificial

<220>
<223>  Reverse primer for analysing, in combination with SEQ ID NO: 9,
       the expression of cyclophilin B by RT-PCR

<400>  10
gcccgtagtg cttcagtttg                                                    20

SEQUENCE LISTING

<110>  Newbiotechnic, S.A.
       Universidad Pablo de Olavide
       University of Stockholm and Huddinge Hospital

<120>  Polyisoperenoid epoxides useful for decreasing cholesterol and/or
       increasing coenzyme Q biosynthesis

<130>  P2919EP00

<160>  10

<170>  PatentIn version 3.3

<210>  1
<211>  20
<212>  DNA
<213>  Artificial

<220>
<223>  Forward primer for analysing, in combination with SEQ ID NO: 2,
       the expression of trans-prenyltransferase (CoQ1) by RT-PCR

<400>  1
tggtccactg aaagacatgc                                                20

<210>  2
<211>  20
<212>  DNA
<213>  Artificial

<220>
<223>  Reverse primer for analysing, in combination with SEQ ID NO: 1,
       the expression of trans-prenyltransferase (CoQ1) by RT-PCR

<400>  2
tccacaacct tggtgttctg                                                20

<210>  3
<211>  20
<212>  DNA
<213>  Artificial

<220>
<223>  Forward primer for analysing, in combination with SEQ ID NO: 4,
       the expression of decaprenyl-4-hydroxybenzoate transferase (CoQ2)
       by RT-PCR

<400>  3
caactagcct tgggcttgac                                                20

<210>  4
<211>  20
<212>  DNA
<213>  Artificial

<220>
<223>  Reverse primer for analysing, in combination with SEQ ID NO: 3,
       the expression of decaprenyl-4-hydroxybenzoate transferase (CoQ2)
       by RT-PCR

<400>  4
atggatcaca ggaacccttg                                                    20


<210>  5
<211>  19
<212>  DNA
<213>  Artificial

<220>
<223>  Forward primer for analysing, in combination with SEQ ID NO: 6,
       the expression of farnesyl-PP synthase (FDPS) by RT-PCR

<400>  5
aggtggctgg gttccctac                                                     19


<210>  6
<211>  21
<212>  DNA
<213>  Artificial

<220>
<223>  Reverse primer for analysing, in combination with SEQ ID NO: 5,
       the expression of farnesyl-PP synthase (FDPS) by RT-PCR

<400>  6
tctggtctcc gttcattctg a                                                  21


<210>  7
<211>  19
<212>  DNA
<213>  Artificial

<220>
<223>  Forward primer for analysing, in combination with SEQ ID NO: 8,
       the expression of squalene synthase by RT-PCR

<400>  7
cagaccagtc gcagtttcg                                                     19


<210>  8
<211>  21
<212>  DNA
<213>  Artificial

<220>
<223>  Reverse primer for analysing, in combination with SEQ ID NO: 7,
       the expression of squalene synthase by RT-PCR

<400>  8
caagggagat cgttgggaag t                                                  21

<210>  9
<211>  20
<212>  DNA
<213>  Artificial

<220>
<223>  Forward primer for analysing, in combination with SEQ ID NO: 10,
       the expression of cyclophilin B by RT-PCR

<400>  9
ggcacaggag gaaagagcat                                                20


<210>  10
<211>  20
<212>  DNA
<213>  Artificial

<220>
<223>  Reverse primer for analysing, in combination with SEQ ID NO: 9,
       the expression of cyclophilin B by RT-PCR

<400>  10
gcccgtagtg cttcagtttg                                                20

SEQUENCE LISTING

<110> Newbiotechnic, S.A.
Universidad Pablo de Olavide
University of Stockholm and Huddinge Hospital

<120> Polyisoperenoid epoxides useful for decreasing cholesterol and/or
increasing coenzyme Q biosynthesis

<130> P2919EP00

<160> 10

<170> PatentIn version 3.3

<210> 1
<211> 20
<212> DNA
<213> Artificial

<220>
<223> Forward primer for analysing, in combination with SEQ ID NO: 2,
the expression of trans-prenyltransferase (CoQ1) by RT-PCR

<400> 1
tggtccactg aaagacatgc 20

<210> 2
<211> 20
<212> DNA
<213> Artificial

<220>
<223> Reverse primer for analysing, in combination with SEQ ID NO: 1,
the expression of trans-prenyltransferase (CoQ1) by RT-PCR

<400> 2
tccacaacct tggtgttctg 20

<210> 3
<211> 20
<212> DNA
<213> Artificial

<220>
<223> Forward primer for analysing, in combination with SEQ ID NO: 4,
the expression of decaprenyl-4-hydroxybenzoate transferase (CoQ2)
by RT-PCR

<400> 3
caactagcct tgggcttgac 20

<210> 4
<211> 20
<212> DNA
<213> Artificial

<220>
<223>  Reverse primer for analysing, in combination with SEQ ID NO: 3,
      the expression of decaprenyl-4-hydroxybenzoate transferase (CoQ2)
      by RT-PCR

<400>  4
atggatcaca ggaacccttg                                                  20


<210>  5
<211>  19
<212>  DNA
<213>  Artificial

<220>
<223>  Forward primer for analysing, in combination with SEQ ID NO: 6,
      the expression of farnesyl-PP synthase (FDPS) by RT-PCR

<400>  5
aggtggctgg gttccctac                                                   19


<210>  6
<211>  21
<212>  DNA
<213>  Artificial

<220>
<223>  Reverse primer for analysing, in combination with SEQ ID NO: 5,
      the expression of farnesyl-PP synthase (FDPS) by RT-PCR

<400>  6
tctggtctcc gttcattctg a                                                21


<210>  7
<211>  19
<212>  DNA
<213>  Artificial

<220>
<223>  Forward primer for analysing, in combination with SEQ ID NO: 8,
      the expression of squalene synthase by RT-PCR

<400>  7
cagaccagtc gcagtttcg                                                   19


<210>  8
<211>  21
<212>  DNA
<213>  Artificial

<220>
<223>  Reverse primer for analysing, in combination with SEQ ID NO: 7,
      the expression of squalene synthase by RT-PCR

<400>  8
caagggagat cgttgggaag t                                                21

<210>  9
<211>  20
<212>  DNA
<213>  Artificial

<220>
<223>  Forward primer for analysing, in combination with SEQ ID NO: 10,
       the expression of cyclophilin B by RT-PCR

<400>  9
ggcacaggag gaaagagcat                                                20


<210>  10
<211>  20
<212>  DNA
<213>  Artificial

<220>
<223>  Reverse primer for analysing, in combination with SEQ ID NO: 9,
       the expression of cyclophilin B by RT-PCR

<400>  10
gcccgtagtg cttcagtttg                                                20